# EUROPEAN PATENT SPECIFICATION

(11) **EP 0 794 774 B1**
(45) Date of publication and mention of the grant of the patent: **12.10.2005**
(21) Application number: 95944363.1
(22) Date of filing: 21.12.1995
(51) Int. Cl.: A61K 31/38, A61K 31/41, A61K 31/435, A61K 31/505, C07D 271/06, C07D 285/12, C07D 333/38, C07D 213/30, C07D 413/04, C07D 239/42, C07D 277/24, C07D 213/38, C07D 213/40, C07D 271/10, C07C 49/753, C07C 43/253

(54) **4,4-(DISUBSTITUTED)CYCLOHEXAN-1-OLS MONOMERS AND RELATED COMPOUNDS**
MONOMERE DER 4,4-(DISUBSTITUIERTEN)CYCLOHEXAN-1-OLE UND VERWANDTE VERBINDUNGEN
MONOMERES DE 4,4-(DISUBSTITUE)CYCLOHEXAN-1-OLES ET COMPOSES APPARENTES

(30) Priority: 23.12.1994 US 363506
(43) Date of publication of application: 17.09.1997
(73) Proprietor: SMITHKLINE BEECHAM CORPORATION, Philadelphia, PA 19102 (US)
(72) Inventor: CHRISTENSEN, Siegfried B., IV, Philadelphia, PA 19103 (US); KARPINSKI, Joseph M., Pottstown, PA 19464 (US); RYAN, M.Dominic, Pottstown, PA 19464 (US); BENDER, Paul E., Cherry Hill, NJ 08003 (US)
(74) Representative: Rutter, Keith
(86) International application number: PCT/US1995/016711
(87) International publication number: WO 1996/019988

(56) References cited:
- WO-A-93/19751
- WO-A-96/38150
- US-A- 5 449 687

## Description

### Field of the Invention

The present invention relates to novel 4,4-(disubstituted)cyclohexan-1-ols monomers and related compounds, pharmaceutical compositions containing these compounds, and their use in treating allergic and inflammatory diseases and for inhibiting the production of Tumor Necrosis Factor (TNF).

### Background of the Invention

Bronchial asthma is a complex, multifactorial disease characterized by reversible narrowing of the airway and hyperreactivity of the respiratory tract to external stimuli.

Identification of novel therapeutic agents for asthma is made difficult by the fact that multiple mediators are responsible for the development of the disease. Thus, it seems unlikely that eliminating the effects of a single mediator will have a substantial effect on all three components of chronic asthma. An alternative to the "mediator approach" is to regulate the activity of the cells responsible for the pathophysiology of the disease.

One such way is by elevating levels of cAMP (adenosine cyclic 3',5'-monophosphate). Cyclic AMP has been shown to be a second messenger mediating the biologic responses to a wide range of hormones, neurotransmitters and drugs; [Krebs Endocrinology Proceedings of the 4th Intanarional Congress Excerpta Medica, 17-29, 1973]. When the appropriate agonist binds to specific cell surface receptors, adenylate cyclase is activated, which converts Mg⁺²-ATP to cAMP at an accelerated rate.

Cyclic AMP modulates the activity of most, if not all, of the cells that contribute to the pathophysiology of extrinsic (allergic) asthma. As such, an elevation of cAMP would produce beneficial effects including: 1) airway smooth muscle relaxation, 2) inhibition of mast cell mediator release, 3) suppression of neutrophil degranulation, 4) inhibition of basophil degranulation, and 5) inhibition of monocyte and macrophage activation. Hence, compounds that activate adenylate cyclase or inhibit phosphodiesterase should be effective in suppressing the inappropriate activation of airway smooth muscle and a wide variety of inflammatory cells. The principal cellular mechanism for the inactivation of cAMP is hydrolysis of the 3'-phosphodiester bond by one or more of a family of isozymes referred to as cyclic nucleotide phosphodiesterases (PDEs).

It has now been shown that a distinct cyclic nucleotide phosphodiesterase (PDE) isozyme, PDE IV, is responsible for cAMP breakdown in airway smooth muscle and inflammatory cells. [Torphy, "Phosphodiesterase Isozymes: Potential Targets for Novel Anti-asthmatic Agents" in New Drugs for Asthma, Barncs, ed. IBC Technical Services Ltd., 1989]. Research indicates that inhibition of this enzyme not only produces airway smooth muscle relaxation, but also suppresses degranulation of mast cells, basophils and neutrophils along with inhibiting the activation of monocytes and neutrophils. Moreover, the beneficial effects of PDE IV inhibitors are markedly potendated when adenylate cyclase activity of target cells is elevated by appropriate hormones or autocoids, as would be the case *in vivo*. Thus PDE IV inhibitors would be effective in the asthmatic lung, where levels of prostaglandin E₂ and prostacyclin (activators of adenylate cyclase) are elevated. Such compounds would offer a unique approach toward the pharmacotherapy of bronchial asthma and possess significant therapeutic advantages over agents currently on the market.

The compounds of this invention also inhibit the production of Tumor Necrosis Factor (TNF), a serum glycoprotein. Excessive or unregulated TNF production has been implicated in mediating or exacerbating a number of diseases including rheumatoid arthritis, rheumatoid spondylitis, osteoarthritis, gouty arthritis and other arthritic conditions; sepsis, septic shock, endotoxic shock, gram negative sepsis, toxic shock syndrome, adult respiratory distress syndrome, cerebral malaria, chronic pulmonary inflammatory disease, silicosis, pulmonary sarcoidosis, bone resorption diseases, reperfusion injury, graft vs. host reaction, allograft rejections, fever and myalgias due to infection, such as influenza, cachexia secondary to infection or malignancy, cachexia secondary to human acquired immune deficiency syndrome (AIDS), AIDS, ARC (AIDS related complex), keloid formation, scar tissue formation, Crohn's disease, ulcerative colitis, or pyresis, in addition to a number of autoimmune diseases, such as multiple sclerosis, autoimmune diabetes and systemic lupus erythematosis.

AIDS results from the infection of T lymphocytes with Human Immunodeficiency Virus (HIV). At least three types or strains of HIV have been identified, i.e., HIV-1, HIV-2 and HIV-3. As a consequence of HIV infection, T-cell-mediated immunity is impaired and infected individuals manifest severe opportunistic infections and/or unusual neoplasms. HIV entry into the T lymphocyte requires T lymphocyte activation. Viruses such as HIV-1 or HIV-2 infect T lymphocytes after T cell activation and such virus protein expression and/or replication is mediated or maintained by such T cell activation. Once an activated T lymphocyte is infected with HIV, the T lymphocyte must continue to be maintained in an activated state to permit HIV gene expression and/or HIV replication.

Cytokines, specifically TNF, are implicated in activated T-cell-mediated HIV protein expression and/or virus replication by playing a role in maintaining T lymphocyte activation. Therefore, interference with cytokine activity such as by inhibition of cytokine production, notably TNF, in an HIV-infected individual aids in limiting the maintenance of T cell activation, thereby reducing the progression of HIV infectivity to previously uninfected cells which results in a slowing or climination of the progression of immune dysfunction caused by HIV infection. Monocytes, macrophages, and related cells, such as kupffer and glial cells, have also been implicated in maintenance of the HIV infection. These cells, like T cells, are targets for viral replication and the level of viral replication is dependent upon the activation state of the cells. [See Rosenberg *et al*., The Immunopathogenesis of HIV Infection, Advances in Immunology, VoL 57, 1989]. Monokines, such as TNF, have been shown to activate HIV replication in monocytes and/or macrophages [See Poli *et al*., Proc. Natl. Acad. Sci., 87:782-784, 1990], therefore, inhibition of monokine production or activity aids in limiting HIV progression as stated above for T cells.

TNF has also been implicated in various roles with other viral infections, such as the cytome galovirus (CMV), influenza virus, adenovirus, and the herpes virus for similar reasons as those noted.

TNF is also associated with yeast and fungal infections. Specifically *Candida albicans* has been shown to induce TNF production *in vitro* in human monocytes and natural killer cells. [See Riipi *et al*., Infection and Immunity, 58(9):2750-54, 1990; and Jafari *et al*., Journal of Infectious Diseases, 164:389-95, 1991. See also Wasan *et al*., Antimicrobial Agents and Chemotherapy, 35,(10):2046-48, 1991; and Luke *et al*., Journal of Infectious Diseases, 162:211-214,1990].

The ability to control the adverse effects of TNF is furthered by the use of the compounds which inhibit TNF in mammals who are in need of such use. There remains a need for compounds which are useful in treating TNF-mediated disease states which are exacerbated or caused by the excessive and/or unregulated production of TNF.

### Summary of the Invention

Compounds of the Formula (I): 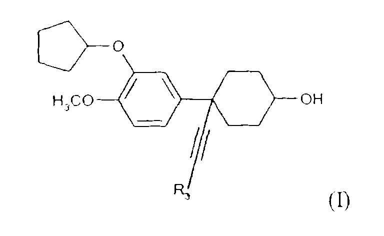 wherein:
R₃ is selected from 4-pyridyl, 3-(5-trifluoromethyl-[1,2,4]oxadiazol-3-yl)phenyl, 3-(5-methyl-[1,3,4]thiadiazol-2-yl)phenyl, 2-acetamidopyrimidin-5-yl, 2-aminopyrimidin-5-yl, and 2-methylaminopyrimidin-5-yl and wherein the compounds of formula (I) are:
   *cis*-[4-(3-cyclopentyloxy-4-methoxyphenyl)-4-(4-pyridylethynyl)-cyclohexan-1-ol];
   *cis*-4-(3-cyclopentyloxy-4-methoxyphenyl)-4-[3-(5-trifluoromethyl-[1,2,4]oxadiazol-3-yl)phenylethynyl]cyclohexan-1-ol;
   *cis*-4-(3-cyclopentyloxy-4-methoxyphenyl)-4-[3-(5-methyl-[1,3,4]thiadiazol-2-yl)phenylethynyl]cyclohexan-1-ol;
   *trans*-[4-(2-acetamidopyrimidin-5-ylethynyl)-4-(3-cyclopentyloxy-4-methoxyphenyl)cyclohexan-1-ol];
   *trans*-[4-(2-aminopyrimidin-5-yl-ethynyl)-4-(3-cyclopentyloxy-4-methoxyphenyl)-cyclohexan-1-ol];
   *cis*-[4-(2-methylaminopyrimidin-5-ylethynyl)-4-(3-cyclopentyloxy-4-methoxyphenyl)cyclohexan-1-ol]. Further compounds are:
      *trans*-[4-(2-aminopyrimidin-5-ylethynyl)-4-(3-cyclopentyloxy-4-methoxyphenyl)cyclohexyl-1-amine], and;
      *cis*-[4-(2-aminopyrimidin-5-ylethynyl)-4-(3-cyclopentyloxy-4-methoxyphenyl)cyclohexyl-1-amine], cyclohexylsulfamate salt;
and the pharmaceutically acceptable salts thereof.

This invention also relates to the pharmaceutical compositions comprising a compound of Formula (I) and a pharmaceutically acceptable carrier or diluent.

The invention also relates to a method of mediation or inhibition of the enzymatic activity (or catalytic activity) of PDE IV in mammals, including humans, which comprises administering to a mammal in need thereof an effective amount of a compound of Formula (I) as shown below.

The invention further provides a method for the treatment of allergic and inflammatory disease which comprises administering to a mammal, including humans, in need thereof, an effective amount of a compound of Formula (I).

The invention also provides a method for the treatment of asthma which comprises administering to a mammal, including humans, in need thereof, an effective amount of a compound of Formula (I).

This invention also relates to a method of inhibiting TNF production in a mammal, including humans, which method comprises administering to a mammal in need of such treatment, an effective TNF inhibiting amount of a compound of Formula (I). This method may be used for the prophylactic treatment or prevention of certain TNF mediated disease states amenable thereto.

This invention also relates to a method of treating a human afflicted with a human immunodeficiency virus (HIV), which comprises administering to such human an effective TNF inhibiting amount of a compound of Formula (I).

Compounds of Formula (I) are also useful in the treatment of additional viral infections, where such viruses are sensitive to upregulation by TNF or will elicit TNF production *in vivo*.

In addition, compounds of Formula (I) are also useful in treating yeast and fungal infections, where such yeast and fungi are sensitive to upregulation by TNF or will elicit TNF production *in vivo*.

### Detailed Description of the Invention

This invention also relates to a method of mediating or inhibiting the enzymatic activity (or catalytic activity) of PDE TV in a mammal in need thereof and to inhibiting the production of TNF in a mammal in need thereof, which comprises administering to said mammal an effective amount of a compound of Formula (I).

Phosphodiesterase IV inhibitors are useful in the treatment of a variety of allergic and inflammatory diseases including: asthma, chronic bronchitis, atopic dermatitis, urticaria, allergic rhinitis, allergic conjunctivitis, vernal conjunctivitis, cosinophilic granuloma, psoriasis, rheumatoid arthritis, septic shock, ulcerative colitis, Crohn's disease, reperfusion injury of the myocardium and brain, chronic glomerulonephritis, endotoxic shock and adult respiratory distress syndrome. In addition, PDE IV inhibitors are useful in the treatment of diabetes insipidus and central nervous system disorders such as depression and multi-infarct dementia.

The viruses contemplated for treatment herein are those that produce TNF as a result of infection, or those which are sensitive to inhibition, such as by decreased replication, directly or indirectly, by the TNF inhibitors of Formula (I). Such viruses include, but are not limited to HIV-1, HIV-2 and HIV-3, cytomegalovirus (CMV), influenza, adenovirus and the Herpes group of viruses, such as, but not limited to, *Herpes zoster* and *Herpes simplex*.

This invention more specifically relates to a method of treating a mammal, afflicted with a human immunodeficiency virus (HIV), which comprises administering to such mammal an effective TNF inhibiting amount of a compound of Formula (I).

The compounds of this invention may also be used in association with the veterinary treatment of animals, other than in humans, in need of inhibition of TNF production. TNF mediated diseases for treatment, therapeutically or prophylactically, in animals include disease states such as those noted above, but in particular viral infections. Examples of such viruses include, but are not limited to feline immunodeficiency virus (FIV) or other retroviral infection such as equine infectious anemia virus, caprine arthritis virus, visna virus, maedi virus and other lentiviruses.

The compounds of this invention are also useful in treating yeast and fungal infections, where such yeast and fungi are sensitive to upregulation by TNF or will elicit TNF production *in vivo*. A preferred disease state for treatment is fungal meningitis. Additionally, the compounds of Formula (I) may be administered in conjunction with other drugs of choice for systemic yeast and fungal infections. Drugs of choice for fungal infections, include but are not limited to the class of compounds called the polymixins, such as Polymycin B, the class of compounds called the imidazoles, such as clotrimazole, econazole, miconazole, and ketoconazole; the class of compounds called the triazoles, such as fluconazole, and itranazole, and the class of compound called the Amphotericins, in particular Amphotericin B and liposomal Amphotericin B.

The compounds of Formula (I) may also be used for inhibiting and/or reducing the toxicity of an anti-fungal, anti-bacterial or anti-viral agent by administering an effective amount of a compound of Formula (I) to a mammal in need of such treatment. Preferably, a compound of Formula (I) is administered for inhibiting or reducing the toxicity of the Amphotericin class of compounds, in particular Amphotericin B.

"Inhibiting the production of IL-1" or "inhibiting the production of TNF" means:
a) a decrease of excessive *in vivo* IL-1 or TNF levels, respectively, in a human to normal levels or below normal levels by inhibition of the *in vivo* release of IL-1 by all cells, including but not limited to monocytes or macrophages;
b) a down regulation, at the translational or transcriptional level, of excessive *in vivo* IL-1 or TNF levels, respectively, in a human to normal levels or below normal levels; or
c) a down regulation, by inhibition of the direct synthesis of IL-1 or TNF levels as a postranslational event.

The phrase "TNF mediated disease or disease states" means any and all disease states in which TNF plays a role, either by production of TNF itself, or by TNF causing another cytokine to be released, such as but not limited to IL-1 or IL-6. A disease state in which IL-1, for instance is a major component, and whose production or action, is exacerbated or secreted in response to TNF, would therefore be considered a disease state mediated by TNF. As TNF-β (also known as lymphotoxin) has close structural homology with TNF-α (also known as cachectin), and since each induces similar biologic responses and binds to the same cellular receptor, both TNF-α and TNF-β are inhibited by the compounds of the present invention and thus are herein referred to collectively as "TNF" unless specifically delineated otherwise. Preferably TNF-α is inhibited.

"Cytokine" means any secreted polypeptide that affects the functions of cells, and is a molecule which modulates interactions between cells in immune, inflammatory, or hematopoietic responses. A cytokine includes, but is not limited to, monokines and lymphokines regardless of which cells produce them.

The cytokine inhibited by the present invention for use in the treatment of a HIV-infected human must be a cytokine which is implicated in (a) the initiation and/or maintenance of T cell activation and/or activated T cell-mediated HIV gene expression and/or replication, and/or (b) any cytokine-mediated disease associated problem such as cachexia or muscle degeneration. Preferrably, his cytokine is TNF-α.

All of the compounds of Formula (I) are useful in the method of inhibiting the production of TNF, preferably by macrophages, monocytes or macrophages and monocytes, in a mammal, including humans, in need thereof. All of the compounds of Formula (I) are useful in the method of inhibiting or mediating the enzymatic or catalytic activity of PDE IV and in treatment of disease states mediated thereby.

Pharmaceutically acceptable salts of the instant compounds, where they can be prepared, are also intended to be covered by this invention. These salts will be ones which are acceptable in their application to a pharmaceutical use.

By that it is meant that the salt will retain the biological activity of the parent compound and the salt will not have untoward or deleterious effects in its application and use in treating diseases.

It will be recognized that some of the compounds of Formula (I) may exist in both racemic and optically active forms; some may also exist in distinct diastereomeric forms possessing distinct physical and biological properties. All of these compounds are considered to be within the scope of the present invention.

Pharmaceutically acceptable salts are prepared in a standard manner. The parent compound, dissolved in a suitable solvent, is treated with an excess of an organic or inorganic acid, in the case of acid addition salts of a base, or an excess of organic or inorganic base where the molecule contains a COOH for example.

Pharmaceutical compositions of the present invention comprise a pharmaceutical carrier or diluent and some amount of a compound of the formula (I). The compound may be present in an amount to effect a physiological response, or it may be present in a lesser amount such that the user will need to take two or more units of the composition to effect the treatment intended. These compositions may be made up as a solid, liquid or in a gaseous form. Or one of these three forms may be transformed to another at the time of being administered such as when a solid is delivered by aerosol means, or when a liquid is delivered as a spray or aerosol.

The nature of the composition and the pharmaceutical carrier or diluent will, of course, depend upon the intended route of administration, for example parenterally, topically, orally or by inhalation.

For topical administration the pharmaceutical composition will be in the form of a cream, ointment, liniment, lotion, pastes, aerosols, and drops suitable for administration to the skin, eye, car, or nose.

For parenteral administration the pharmaceutical composition will be in the form of a sterile injectable liquid such as an ampule or an aqueous or non-aqueous liquid suspension.

For oral administration the pharmaceutical composition will be in the form of a tablet, capsule, powder, pellet, atroche, lozenge, syrup, liquid, or emulsion.

When the pharmaceutical composition is employed in the form of a solution or suspension, examples of appropriate pharmaceutical carriers or diluents include: for aqueous systems, water, for non-aqueous systems, ethanol, glycerin, propylene glycol, corn oil, cottonseed oil, peanut oil, sesame oil, liquid parafins and mixtures thereof with water, for solid systems, lactose, kaolin and mannitol; and for acrosol systems, dichlorodifluoromethane, chlorotrifluoroethane and compressed carbon dioxide. Also, in addition to the pharmaceutical carrier or diluent, the instant compositions may include other ingredients such as stabilizers, antioxidants, preservatives, lubricants, suspending a gents, viscosity modifiers and the like, provided that the additional ingredients do not have a detrimental effect on the therapeutic action of the instant compositions.

The pharmaceutical preparations thus described are made following the conventional techniques of the pharmaceutical chemist as appropriate to the desired end product.

In these compositions, the amount of carrier or diluent will vary but preferably will be the major proportion of a suspension or solution of the active ingredient. When the diluent is a solid it may be present in lesser, equal or greater amounts than the solid active ingredient.

Usually a compound of formula I is administered to a subject in a composition comprising a nontoxic amount sufficient to produce an inhibition of the symptoms of a disease in which leukotrienes are a factor. Topical formulations will contain between about 0.01 to 5.0% by weight of the active ingredient and will be applied as required as a preventative or curative agent to the affected area. When employed as an oral, or other ingested or injected regimen, the dosage of the composition is selected from the range of from 50 mg to 1000 mg of active ingredient for each administration. For convenience, equal doses will be administered 1 to 5 times daily with the daily dosage regimen being selected from about 50 mg to about 5000 mg

No unacceptable toxicological effects are expected when these compounds are administered in accordance with the present invention.

### Methods Of Preparation

### Synthetic Scheme(s) With Textual Description

Compounds of Formula (I) may be prepared by the processes disclosed herein which comprise reacting a terminal acetylene, wherein Z represents Z as defined in relation to Formula (I) or a group convertible to Z, as, *e*.*g*., compound 1-Scheme 1, with an appropriate halide, R₃X, wherein R₃ represents R₃ as defined in relation to Formula (I) or a group convertible to R₃, in the presence of a suitable catalyst, such as a copper (I) halide and a bivalent or zerovalent palladium compound in the presence of, *e*.*g*., triphenylphosphine, in a suitable solvent, such as an amine, as in the pr° Cedure of Brandsma *et al*. (Syn. Comm., 1990, 20. 1889), provides a compound of the Formula 2-Scheme 1. Compounds of the Formula 1-Scheme 1 may be prepared by pr° Cedures analogous to those described in copending U.S. application 07/862111, 07/968761 and PCT application number PCT/US93/02516 published under WIPO publication number WO93/19751.

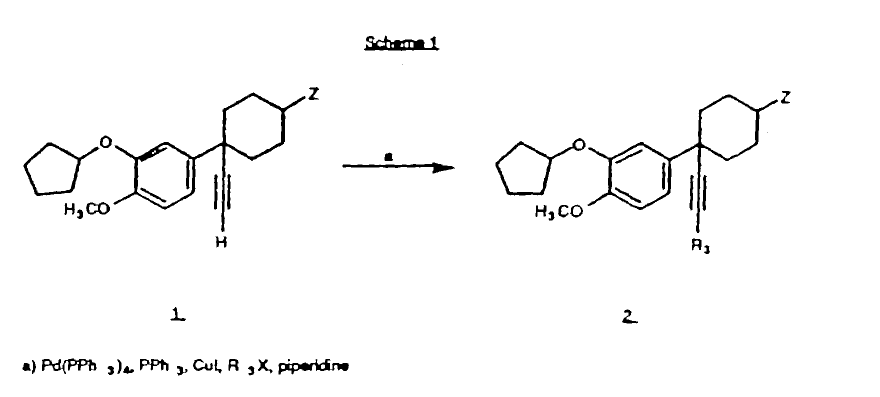

Alternatively, compounds of the Formula (I), wherein Z and R₃ represent Z and R₃ as defined in relation to Formula (I) or a group convertible to Z or R₃, may be prepared from the corresponding ketones as, *e*.*g*., compound 1-Scheme 2, by the synthetic pr° Cedures described in PCT application number PCT/US93/01990 and PCT/US93/02325 published as WIPO publication number WO93/19750.

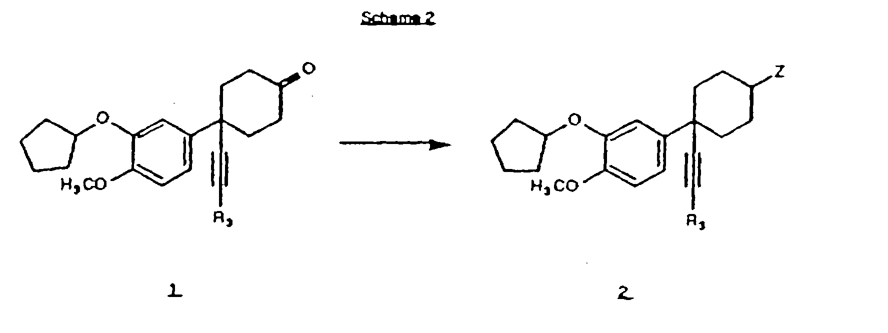

Alternatively, oxidative carbonylation of a terminal acetylene as, *e*.*g*., compound 1-Scheme 3, using an appropriate metal salt, such as a copper salt with a catalytic amount of a palladium salt, in the presence of a suitable base as an acid trap, such as sodium acetate, in a suitable alcohol, such as methanol, as in the method of Tsuji *et al*. (Tet. Lett., 1980, 21, 849), then provides the compound of the Formula 2-Scheme 3; such compounds may then be converted to other compounds of the Formula (I) by manipulation of the ketone as described above and by independent manipulation of the carboxylic ester moiety using standard transesterification or amidation conditions. Syntheses of such ketone starting materials are described in published PCT application PCT/US93/02045 (WIPO publication number WO 93/19748) or published PCT application PCT/US93/02325 (WIPO publication number WO/93/19750).

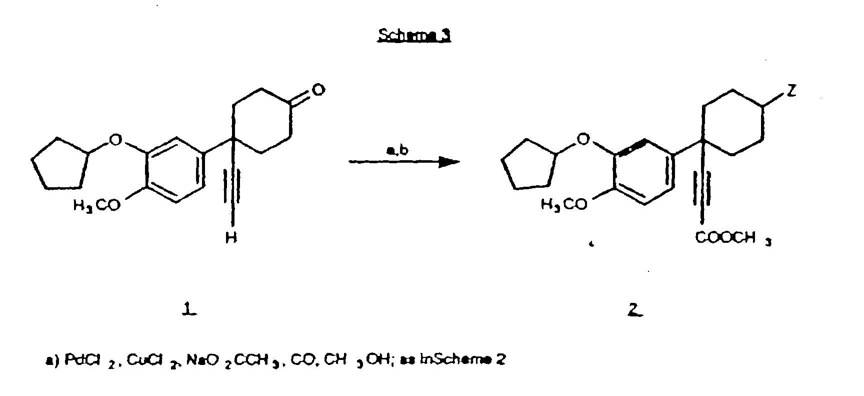

Likewise, oxidative carbonylation of a terminal acetylene as, *e*.*g*., compound 1-Scheme 4, wherein Z represents Z as defined in relation to Formula (I) or a group convertible to Z, using an appropriate metal salt, such as a copper salt with a catalytic amount of a palladium salt, in the presence of a suitable base as an acid trap, such as sodium acetate, in a suitable alcohol, such as methanol, as in the method of Tsuji *et al*. (Tet. Lett, 1980, 21, 849), then provides the compound of the Formula 2-Scheme 4; such compounds may then be converted to other compounds of the Formula (I) by manipulation of the carboxylic ester moiety using standard transesterification or amidation conditions.

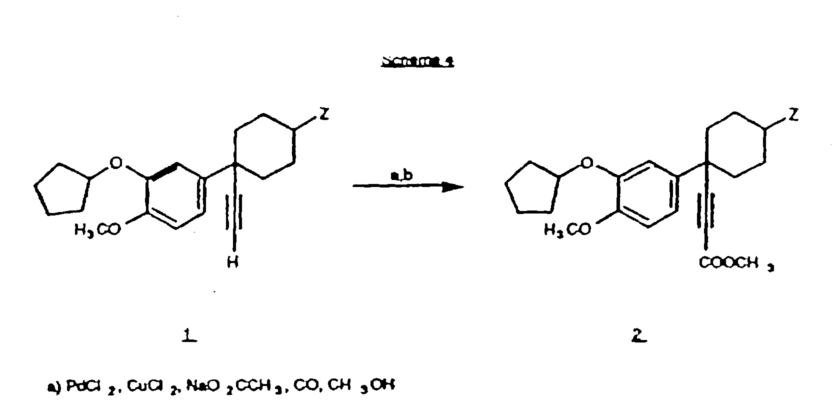

Compounds where Z is a group other than -OH can be prepared by methods known in the art and in particular by manipulation of the -OH. Such methods are described in co-pending U.S. application 07/968753 and PCT application serial number PCT/US93/02325 (WIPO publication number WO/93/19750).

Preparation of the remaining compounds of the Formula (I) may be accomplished by procedures analogous to those described above and in the Examples, *infra*.

It will be recognized that some compounds of the Formula (I) may exist in distinct diastereomeric forms possessing distinct physical and biological properties; such isomers may be separated by standard chromatographic methods.

The following examples are given to further illustrate the described invention. These examples are intented solely for illustrating the invention and should not be read to limit the invention in any manner. Reference is made to the claims for what is reserved to the inventors hereunder.

### Experimentals

### Example 1

### Preparation of cis-[4-(3-cyclopentyloxy-4-methoxyphenyl)-4-(4-pyridylethynyl)-cyclohexan-1-ol]

### 1a) trans-[4-(3-cyclopentyloxy-4-methoxyphenyl)-4-ethynylcyclohexan-1-ol] and cis-[4-(3-cyclopentyloxy-4-methoxyphenyl)-4-ethynylcyclohexan-1-ol]

To a solution of 4-(3-cyclopentyloxy-4-methoxyphenyl)-4-ethynylcyclohexan-1-one (0.34 g. 1.1 mmol, prepared as described in published PCT application PCT/US93/001990 (WIPO publication number WO 93/19748) of PCT/US93/02325 (WIPO publication number WO/93/19750) in 1,2-dimethoxyethane (5 mL) under an argon atmosphere was added sodium borohydride (0.08 g, 2.2 mmol) and the mixture was stirred at room temperature for 0.5 h. Water was added, the mixture was extracted three times with dichloromethane, was dried (magnesium sulfate) and was evaporated. Purification by flash chromatography, cluting with 3:7 ethyl acetate:hexanes, provided *trans*-[4-(3-cyclopentyloxy-4-methoxyphenyl)-4-ethynylcyclohexan-1-ol], (described in U. S. Patent application in PCT application number PCT/US93/02516 published under WIPO publication number WO93/19751_as *cis*-[4-(3-cyclopentyloxy-4-methoxyphenyl)-4-ethynylcyclohexan-1-ol]) as a wax. ¹H-NMR (400 MHz, CDCl₃) δ 7.14 (d, J=2.3 Hz, 1H), 7.04 (dd, J=8.5, 2.3 Hz, 1H), 6.82 (d, J=8.5 Hz, 1H), 4.79 (m, 1H), 3.83 (s. 3H), 3.68 (m, 1H), 2.46 (s, 1H), 1.7 - 2.1 (m, 14H), 1.6 (m, 2H).

*cis*-[4-(3-cyclopentyloxy-4-methoxyphenyl)-4-ethynylcyclohexan-1-ol] also was isolated from this pr° Cedure as a colorless oiL ¹H-NMR (400 MHz, CDCl₃) δ 7.17 (d, J=1.9 Hz, 1H), 7.10(d, J=8.4 Hz, 1H), 6.84 (d, J=8.4 Hz, 1H), 4.80 (m, 1H), 4.13 (br s, 1H), 3.84 (s, 3H), 2.38 (s, 1H), 2.15 (m, 4H), 1.7 - 2.0 (m, 10H), 1.75 (m, 2H).

### 1b) cis-[4-(3-cyclopentyloxy-4-methoxyphenyl)-4-(4-pyridylethynyl)cyclohexan-1-ol]

To a solution of *trans*-[4-(3-cyclopentyloxy-4-methoxyphenyl)-4-ethynylcyclohexan-1-ol] (0.15 g, 0.48 mmol) and 4-bromopyridine (0.75g, 5 mmol) in piperidine (2 mL) under an argon atmosphere were added tetrakis(triphenylphosphine)-palladium(0) (0.022 g, 4%), copper (I) iodide (0.005 g. 6%) and a small crystal of triphenylphosphine, and the mixture was heated at 80-85° C for 0.5 h. Ammonium chloride was added and the mixture was extracted three times with dichloromethane, the extract was dried (magnesium sulfate) and was evaporated. Purification by flash chromatography, cluting with 3:1 ethyl acetate/hexanes, provided *cis*-[4-(3-cyclopentyloxy-4-methoxyphenyl)-4-(4-pyridylethynyl)-cyclohexan-1-ol] as a pale yellow solid, which was triturated from ether-hexanes, mp 183 - 184° C. ¹H-NMR (400 MHz. CDCl₃) δ 8.6 (br, 2H), 7.35 (m, 2H), 7.14 (d, J=2 Hz, 1H), 7.06 (dd, J=8.5, 2Hz, 1H), 6.85 (d, J=8.5Hz, 1H), 4.79 (m, 1H), 3.85 (s, 3H), 3.7 (m, 1H), 1.8 - 2.1 (m, 14H), 1.6 (m, 2H).

### Reference Example 2

### cis-[4-(3-cyclopentyloxy-4-methoxyphenyl)-4-(pyrid-2-ylethynyl)cyclohexan-1-ol]

To a solution of *trans*-[4-(3-cyclopentyloxy-4-methoxyphenyl)-4-ethynylcyclohexan-1-ol] (0.14 g, 0.43 mmol) and 2-bromopyridine (0.40 mL, 4.3 mmol) in piperidine (2 mL) under an argon atmosphere were added tetrakis(triphenylphosphine)palladium(0) (0.020 g, 4%), copper(I) iodide (0.005 g, 6%) and a small crystal of triphenylphosphine, and the mixture was heated at 80-85° C for 0.5 h. Ammonium chloride was added and the mixture was extracted three times with dichloromethane, was dried (magnesium sulfate) and was evaporated. Purification by flash chromatography, eluting with 75:25 ethyl acetate:hexanes, provided *cis*-[4-(3-cyclopentyloxy-4-methoxyphenyl)-4-(pyrid-2-ylethynyl)cyclohexan-1-ol] as a white solid (0.14 g, 84%), mp 49-51° C. Anal. (C₂₅H₂₉NO₃·0.5 H₂O) calcd: C, 74.97; H, 7.55; N, 3.50; found: C, 74.90; H, 7.52; N, 333.

### Reference Example 3

### Resolution of (+/-)-3-(3-cyclopentyloxy-4-methoxyphenyl)-3-ethynylcyclohexan-1-one

The compound from Example 1b was resolved in the following manner to give enantiomeric oils: HPLC Rt a 15.5 min (enantiomer 1 = E1), 23.2 min (enantiomer 2 = E2) (Diacel Chiralpak AS®; 21.2 x 250 mm; hexane:isopropanol, 4:1; 10 mL/min; UV detection at 295 nm).

### Reference Example 4

### Preparation of (+/-) 3-(3-cyclopentyloxy-4-methoxyphenyl)-3-phenylethynylcyclohexan-1-one

To a solution of the compound of Example 1b (0.125 g, 0.4 mmol) and iodobenzene (0.4 mL, 2.0 mmol) in piperidine (6 mL) under an argon atmosphere was added trace tetrakis(triphenylphosphine)palladium(0), copper(I) iodide and triphenylphosphine. The mixture was refluxed for 5 h, then conceatrated *in vacuo*. The residue was diluted with ethyl acetate (100 mL), was washed with brine, was dried (M_{g}SO₄) and was evaporated. Purification by flash chromatography, eluting with 2:1 hexanes/ethyl acetate, followed by trituration from ether/hexanes, provided the title compound as white solid (0.09 g, 58%), m.p. 90-91° C.

### Reference Example 5

### Preparation of (+/-) 3-(3-cyclopentyloxy-4-methoxyphenyl)-3-(3-carbomethoxyphenyl)ethynylcyclohexan-1-one

### 5a) methyl 3-iodobenzoate

Methyl 3-iodobenzoate was prepared by standard chemistry well known to those versed in the art and is a white solid, m.p. 40-41° C.

### 5b) (+/-) 3-(3-cyclopentyloxy-4-methoxyphenyl)-3-(3-carbomethoxyphenyl)ethynylcyclohexan-1-one

To a solution of the compound from Example 1b (0.30 g, 0.96 mmol) and methyl 3-iodobenzoate (0.30 g, 1.15 mmol) in triethylamine (10 mL) under an argon atmosphere was added trace tetrakis(triphenylphosphine)palladium(0), copper(I) iodide and triphenylphosphine. The mixture was refluxed for 0.5 h and was then concentrated *in vacuo*. The residue was partitioned between water and ethyl acetate. The organic phase was dried (Na₂SO₄) and was evaporated. Purification by flash chromatography, cluting with 2:1 hexanes/ethyl acetate, provided the title compound as a pale yellow oil (0.35 g, 80%). Anal (C₂₈H₃₀O₅·1.0 H₂O) calcd: C, 72.39; H, 6.94; found: C, 72.47; H, 6.80.

### Reference Example 6

### Preparation of (+/-) 3-(3-carboxyphenylethynyl)-3-(3-cyclopentyloxy-4-methoxyphenyl)cyclohexan-1-one

To solution of the compound from Example 5(b) in 5:5:2 THF/methanol/water (10 mL) under an argon atmosphere was added sodium hydroxide (0.60 g, 1.5 mmol). The mixture was heated at 60° C for 2 h and was then concentrated *in vacuo*. The residue was extracted from 3N HCl with ethyl acetate, was washed with brine, was dried (M_{g}SO₄) and was evaporated. Purification by flash chromatography, eluting with 98:2:0.3 chloroform/methanol/acetic acid, provided a white solid, m.p. 71-73°C.

### Reference Example 7

### Preparation of 3-(3-cyclopentyloxy-4-methoxyphenyl)-3[3-(5-methyl-[1,3,4]thiadiazol-2-yl)phenylethynyl]cyclohexan-1-one

### 7a) 1-iodo-3-(5-methyl-[1,3,4]thiadiazol-2-yl)benzene

1-Iodo-3-(5-methyl-[1,3,4]thiadiazol-2-yl)benzene was prepared by standard chemistry well known to those versed in the art and is white solid, m.p. 86-89° C,

### 7b) 3-(3-cyclopentyloxy-4-methoxyphenyl)-3-[3-(5-methyl-[1,3,4]thiadiazol-2-yl)phenylethynyl]cyclohexan-1-one

To a solution of the compound from Example 3 (E1) (0.10 g, 0.32 mmol) and 1-iodo-3-(5-methyl[1,3,4]thiadiazol-2-yl)benzene (0.10 g, 0.32 mmol) in triethylamine (5 mL) under an argon atmosphere was added trace tetrakis(triphenylphosphine)palldium(0), copper(I) iodide and triphenylphosphine. The mixture was refluxed for 0.20 h, was cooled to room temperature and was concentrated *in vacuo*. The residue was partitioned between ethyl acetate and water. The organic phase was washed with brine, was dried (M_{g}SO₄) and was evaporated. Purification by flash chromatography, eluting with 1:1 hexanes/ethyl acetate provided the title compound as a white solid (0.135 g, 87%), m.p. 97-99° C.

The enantiomer was prepared in a similar manner, starting with the compound from Example 3 (E2), as a white solid, m.p. 97-99° C.

### Reference Example 8

### Preparation of 3-(3-cyclopentyloxy-4-methoxyphenyl)-3-[3-(5-methyl-[1,3,4]oxadiazol-2-yl)phenylethynyl]cyclohexan-1-one

### 8a) 1-Iodo-3-(5-methyl[1,3,4]oxadizol-2-yl)benzene

1-Iodo-3-(5-methyl[1,3,4]oxadiazol-2-yl)benzene was prepared by standard chemistry well known to those versed in the art and is a white solid, m.p. 104-10.5° C.

### 8b) 3-(3-cyclopentyloxy-4-methoxyphenyl)-3-[3-(5-methyl[1,3,4]oxadiazol-2-yl)phenylethynyl]cyclohexan-1-one

To a solution of the compound from Example 3 (0.125 g. 0.4 mmol) and 1-iodo-3-(5-methyl-[1,3,4]oxadiazol-2-yl)benzene (0.09 g, 0.32 mmol) in triethylamine (3 mL) under an argon atmosphere was added trace tetrakis(triphenylphosphine)-palladium(0), copper(I) iodide and triphenylphosphine. The mixture was heated at 80° C for 0.2 h , was cooled to room temperature and was concentrated *in vacuo.* The residue was partitioned between ethyl acetate and water. The organic phase was washed with brine, was dried (M_{g}SO₄) and was evaporated. Purification by flash chromatography, cluting with 2:1 ethyl acetate/hexanes, followed by recrystallization from ethyl acetate/hexanes, provided the title compound as a white solid (0.11 g, 61%), m.p. 117-119° C.

The enantiomer was prepared in a similar manner, starting with the compound from Example 3 (E2), as a white solid, m.p. 117-119° C.

### Reference Example 9

### Preparation of 3-(3-cyclopentyloxy-4-methoxyphenyl)-3-[3-(3-methyl-[1,2,4]oxadiazol-5-yl)phenylethynyl]cyclohexan-1-one

### 9a) 1-iodo-3-(3-methyl-[1,2,4]oxadiazol-5-yl)benzene

1-Iodo-3-(3-methyl-[1,2,4]oxadiazol-5-yl)benzene was prepared by standard chemistry well known to those versed in the art and is a white solid, m.p. 101.5-103° C.

### 9b) 3-(3-cyclopentyloxy-4-methoxyphenyl)-3-[3-(3-methyl-[1,2,4]oxadiazol-5-yl)phenylethynyl]cyclohexan-1-one

To a solution of the compound from Example 3 (0.125 g, 0.4 mmol) and 1-iodo-3-(3-methyl-[1,2,4]oxadiazol-5-yl)benzene (0.09 g, 0.32 mmol) in triethylamine (3mL) under an argon atmosphere was added trace tetrakis(triphenylphosphine)-palladium(0), copper(I) iodide and triphenylphosphine. The mixture was heated at 80° C for 0.2 h, was cooled to room temperature and was concentrated *in vacuo*. The residue was partitioned between ethyl acetate and water. The organic phase was washed with brine, was dried (M_{g}SO₄) and was evaporated. The residue was purified by flash chromatography, eluting with 2:1 hexanes/ethyl acetate, followed by trituration from hexanes/ethyl acetate, to provide the title compound as a white solid, m.p. 122 123° C.

The enantiomer was prepared in a similar manner, starting with the compound from Example 3 (E2), as awhite solid, m.p. 122-123° C.

### Reference Example 10

### Preparation of 3-(3-cyclopentyloxy-4-methoxyphenyl)-3-[3-(5-methyl-[1,2,4]oxadiazol-3-yl)phenylethynyl]cyclohexan-1-one

### 10a) 1-iodo-3-(5-methyl-[1,2,4]oxadiazol-3-yl)benzene

1-iodo-3-(5-methyl-[1,2,4]oxadiazol-3-yl)benzene was prepared by standard chemistry well known to those versed in the art and is a white solid, m.p. 86-87° C.

### 10b) 3-(3-cyclopentyloxy-4-methoxyphenyl)-3-[3-(5-methyl-[1,2,4]oxadiazol-3-yl)phenylethynyl]cyclohexan-1-one

To a solution of the compound from Example 3 (0.125 g, 0.4 mmol) and 1-iodo-3-(5-methyl-[1,2,4]oxadiazol-3-yl)benzene (0.09 g, 0.32 mmol) in triethylamine (3 mL) under an argon atmosphere was added trace tetrakis(triphenylphosphine)-palladium(0), copper(I) iodide and triphenylphosphine. The mixture was heated at 80° C for 0.2 h, was cooled to room temperature and was concentrated *in vacuo*. The residue was partitioned between ethyl acetate and water. The organic phase was washed with brine, was dried (M_{g}SO₄) and was evaporated. Purification by flash chromatography, eluting with 2:1 hexanes/ethyl acetate, followed by trituration from hexanes/ethyl acetate, provided the title compound as colorless crystals (0.12 g, 67%), m.p. 116-118° C.

The enantiomer was prepared in a similar manner, starting with the compound from Example 3 (E2), as colorless crystals, m.p.116-118° C.

### Reference Example 11

### Preparation of 3-(3-cyclopentyloxy-4-methoxyphenyl)-3-(3-cyanophenylethynyl)cyclohexan-1-one

To a solution of the compound from Example 3 (E1) (0.125 g, 0.4 mmol) and 3-iodobenzonitrile (Transworld, 0.09 g, 0.4 mmol) in triethylamine (3mL) under an argon atmosphere was added trace tetrakis(triphenylphosphine)palladium(0), copper(I) iodide and triphenylphosphine. The mixture was heated at 80° C for 0.2 h, was cooled to room temperature and was concentrated *in vacuo*. The residue was partitioned between ethyl acetate and water. The organic phase was washed with brine, was dried (M_{g}SO₄) and was evaporated. The residue was purified by flash chromatography, cluting with 2:1 hexanes/ethyl acetate, to provide the title compound as a clear yellow glass (0.12 g, 73%). MS(EI) m/e 414 [M+H]⁺.

The enantiomer was prepared in similar manner, starting with the compound from Example 3(E2), as a clear yellow glass.

### Reference Example 12

### Preparation of 3-(3-cyclopentyloxy-4-methoxyphenyl)-3-(3-nitrophenylethynyl)cyclohexan-1-one

To a solution of the compound from Example 3 (E1) (0.2 g, 0.64 mmol) and 3-iodonitrobenzene (Aldrich, 0.16 g, 0.64 mnool) in triethylamine (4 mL) under an argon atmosphere was added trace tetrakis(triphenylphosphine)palladium(0), copper(I) iodide and mphenytphosphine. The mixture was heated at 80° C for 0.2 h, was cooled to room temperature and was concentrated *in vacuo*. The residue was partitioned between ethyl acetate and water. The organic phase was washed with brine, was dried (M_{g}SO₄) and was evaporated. Purification by flash chromatography, eluting with 3:1 hexanes/ethyl acetate, provided the title compound as yellow solid (0.25 g, 90%), m.p. 46-48° C.

The enantiomer was prepared in a similar manner, starting with the compound from Example 3 (E2), as a yellow solid. m.p. 46-48° C.

### Reference Example 13

### Preparation of 3-(3-cyclopentyloxy-4-methoxyphenyl)-3-(2-hydroxyethoxyphenylethynyl)cyclohexan-1-one

### 13a) 2-hydroxyethoxy-1-iodobenzene

2-hydroxyethoxy-1-iodobenzene was prepared by standard chemistry well known to those versed in the art and is a colorless oil. ¹H NMR(400 MHz, CDCl₃) δ 7.77 (dd, J=7.9, 13 Hz. 1H), 7.3 (t, J=7H, 1H), 6.84 (d, J=7.9 Hz, 1H), 6.74 (t, J=7.9 Hz, 1H), 4.13 (t, J=4.3 Hz, 2H), 3.99 (t, J=4.3 Hz, 2H), 2.2 (br s, 1H).

### 13b) 3-(3-cyclopentyloxy-4-methoxyphenyl)-3-(2-hydroxyethoxyphenylethynyl)-cyclohexan-1-one

To a solution of the compound from Example 3 (E1) (0.25 g, 0.8 mmol) and 2-hydroxyethoxy-1-iodobenzene (0.21 g, 0.8 mmol) in triethylamine (5 mL) under an argon atmosphere was added trace tetrakis(triphenylphosphine)palladium(0), copper(I) iodide and triphenylphosphine. The mixture was heated at 80° C for 1 h, was cooled to room temperature and was concentrated *in vacuo*. The residue was partitioned between ethyl acetate and water. The organic phase was washed with brine, was dried (M_{g}SO₄) and was evaporated. Purification by flash chromatography, cluting with 1:1 hexanes/ethyl acetate, provided the title compound as a white solid (0.05 g, 14%), m.p. 93-94° C.

### Reference Example 14

### Preparation of 3-(3-acetamidophenylethynyl)-3-(3-cyclopentyloxy-4-methoxyphenyl)cyclohexan-1-one

### 14a) 3-acetamido-1-iodobenzene

3-acetamido-1-iodobenzene was prepared by standard chemistry well known to those versed in the art and is a white solid, m.p. 117-118°C.

### 14b) 3-(3-acetamidophenylethynyl)-3-(3-cyclopentyloxy-4-methoxyphenyl)cyclohexan-1-one.

To a solution of the compound from Example 3 (E1) (0.2 g, 0.64 mmol) and 3-acetamido-1-iodobenzene (0.17 g, 0.64 mmol) in triethylamine (5 mL) under an argon atmosphere was added trace tetrakis(triphenylphosphine)palladium(0), copper(I) iodide and triphenylphosphine. The mixture was heated at 80° C for 0.3 h, was cooled to room temperature and was concentrated *in vacuo*. The residue was purified by flash chromatography, eluting with 1:1 hexanes/ethyl acetate, to provide the title compound as tan solid (0.17 g, 60%), m.p. 58-60° C.

### Reference Example 15

### Preparation of 3-(3-cyclopentyloxy-4-methoxyphenyl)-3-(3-methanesulfonamidophenylethynyl)cyclohexan-1-one

### 15a) 1-iodo-3-methanesulfonamidobenzene

1-iodo-3-methanesulfonamidobenzene was prepared by standard chemistry well known to those versed in the art and is light-pink solid, m.p. 102-103° C.

### 15b) 3-(3-cyclopentyloxy-4-methoxyphenyl)-3-(3-methanesulfonamidophenylethynyl)cyclohexan-1-one

To a solution of the compound from Example 3 (E1) (0.2 g, 0.64 mmol) and 1-iodo-3-methanesulfonamidobenzene (0.19 g, 0.64 mmol) in triethylamine (5 mL) under an argon atmosphere was added trace tetrakis(triphenylphosphine)palladium(0), copper(I) iodide and triphenylphosphine. The mixture was heated at 80° C for 0.3 h, was cooled to room temperature and was concentrated *in vacuo*. The residue was purified by flash chromatography, eluting with 1:1 hexanes/ethyl acetate, to provide the title compound as a tan solid (0.18 g. 58%), m.p. 59-62° C.

### Reference Example 16

### Preparation of 3-(3-aminophenylethynyl)-3-(3-cyclopentyloxy-4-methoxyphenyl)cyclohexan-1-one

### 16a) 1-iodo-3-trifluoroacetamidobenzene

1-iodo-3-trifluoroacetamidobenzene was prepared by standard chemistry well known to those versed in the art and is a white solid, m.p. 120-121° C.

### 16b) 3-(3-cyclopentyloxy-4-methoxyphenyl)-3-(3-trifluoroacetamidophenylethynyl)-cyclohexan-1-one

To a solution of the compound from Example 3 (E1) (0.5 g. 1.6 mmol) and 1-iodo-3-trifluoroacetamidobenzene (0.5 g, 1.6 mmol) in triethylamine (10 mL) under an argon atmosphere was added a small amount of tetrakis(triphenylphosphine)palladium(0), copper(I) iodide and triphenylphosphine. The mixture was heated at 80° C for 0.2 h, was cooled to room temperature and was concentrated *in vacuo.* The residue was purified by flash chromatography, cluting with 3:1 hexanes/ethyl acetate, to give the title compound as a pale yellow solid (0.62 g, 78%), m.p. 63-65° C.

### 16c) 3-(3-aminophenylethynyl)-3-(3-cyclopentyloxy-4-methoxyphenyl)cyclohexan-1-one

To a solution of 3-(3-cyclopentyloxy-4-methoxyphenyl)-3-(3-trifluoroacetamidophenylethynyl)cyclohexan-1-one (0.62 g, 1.24 mmol) in 95:5 methanol/water (10 mL) under an argon atmosphere was added potassium carbonate (0.86 g, 6.2 mmol). The mixture was refluxed for 6 h and was stirred for 18 h at room temperature. The solid precipitate was collected and purified by trituration from ethyl acetate/hexanes to provide the title compound as a white solid (0.39 g, 77%), m.p. 100-102°C.

### Reference Example 17

### cis-[4-(4-cyanothien-2-ylethynyl)-4-(3-cyclopentyloxy-4-methoxyphenyl)-cyclohexan-1-ol]

### 17a) 2-bromo-4-cyanothiophene

2-Bromo-5-cyanothiophene was prepared by standard chemistry well known to those versed in the art and is a colorless oiL ¹H-NMR (400 MHz, CDCl₃) δ 7.85 (d, J=2.2Hz, 1H), 7.25 (d, J=2.2 Hz, 1H) ppm.

### 17b) cis-[4-(4-cyanothien-2-ylethynyl)-4-(3-cyclopentyloxy-4-methoxyphenyl)-cyclohexan-1-ol]

To a solution of *trans*-[4-(3-cyclopentyloxy-4-methoxyphenyl)-4-ethynylcyclohexan-1-ol] (0.20 g, 0.64 mmol) and 2-bromo-4-cyanothiophene (0.12 g, 0.64 mmol) in triethylamine (5 mL) under an argon atmosphere were added tetrakis(triphenyl-phosphine)palladium(0) (0.030 g, 4%), copper(I) iodide (0.008 g, 6%), and a small crystal of triphenylphosphine, and the mixture was heated at 80-85° C for 0.5 h. Hydr° Chloric acid (5 %) was added and the mixture was extracted three times with dichloromethane, was dried (magnesium sulfate) and was evaporated. Purification by flash chromatography, cluting with 1:1 ethyl acetate:hexanes, followed by trituration from dichloromethane-hexanes, provided *cis*-[4-(4-cyanothien-2-ylethynyl)-4-(3-cyclopentyloxy-4-methoxyphenyl)cyclohexan-1-ol] as a pale yellow solid (0.12 g, 45%), mp 70-71° C. Anal. (C₂₅H₂₇NO₃S) calcd: C, 71.23; H, 6.46; N. 3.30; found: C, 71.24; H, 6.72; N, 3.14.

### Reference Example 18

### cis-[4-(3-cyclopentyloxy-4-methoxyphenyl)-4-[4-(5-methyl-[1,2,4]oxadiazol-2-yl)thien-2-ylethynyl] cyclohexan-1-ol]

### 18a) 2-bromo-4-(5-methyl-[1,2,4]oxadiazol-2-yl)thiophene

2-Bromo-4-(5-methyl-[1,2,4]oxadiazol-2-yl)thiophene was prepared by standard chemistry well known to those versed in the art and is a white solid, mp 72-73°C.

### 18b) cis-[4-(3-cyclopentyloxy-4-methoxyphenyl)-4-[4-(5-methyl-[1,2,4]oxadiazol-2-yl)thien-2-ylethynyl]cyclohexan-1-ol]

To a solution of *trans*-[4-(3-cyclopentyloxy-4-methoxyphenyl)-4-ethynylcyclohexan-1-ol] (0.25 g, 0.8 mmol) and 2-bromo-4-(5-methyl-[1,2,4]oxadiazol-2-yl)thiophene (0.20 g. 0.8 mmol) in triethylamine (5 mL) under an argon atmosphere were added tetrakis(triphenylphosphine)palladium(0) (0.038 g, 4%), copper(I) iodide (0.009 g. 6%) and a small crystal of triphenylphosphine, and the mixture was heated at 70-75° C for 0.5 h. Hydrchloric acid (5%) was added and the mixture was extracted three times with dichloromethane, was dried (magnesium sulfate) and was evaporated. Purification by flash chromatography, eluting with 1:1 ethyl acetate:hexanes, provided *cis* [4-(3-cyclopentyloxy-4-methoxyphenyl)-4-[4-(5-methyl-[1,2,4]oxadiazol-2-yl)thien-2-ylethynyl]cyclobexan-1-ol], which was further triturated from dichloromethane-hexanes to give a white solid (0.20 g, 53%), mp 142-143° C. Anal. (C₂₇H₃₀N₂O₄S · 0.75 H₂O) calcd: C, 65.90; H, 6.45; N, 5.69; found: C, 66.06; H, 6.42; N, 5.50

### Reference Example 19

### cis-[4-(2-aminopyrimidin-4-ylethynyl)-4-(3-cyclopentyloxy-4-methoxyphenyl)-cyclohexan-1-ol]

### 19a) 4-iodo-2-thiomethylpyrimidine

4-Iodo-2-thiomethylpyrimidine was prepared following a literature procedure (AJ. Majecd, Ø. Antonsen, T. Benneche, K. Undheim. *Tetrahedron* 1989, *45*, 993-1006).

### 19b) cis-[4-(3-cyclopentyloxy-4-methoxyphenyl)-4-(2-methylthiopyrimidin-4-ylethynyl) cyclohexan-1-ol]

To a solution of *trans*-[4-(3-cyclopentyloxy-4-methoxyphenyl)-4-ethynylcyclohexan-1-ol] (0.30 g, 0.95 mmol) and and 4-iodo-2-thiomethylpyrimidine(0.50 g, 2.5 mmol, as a mixture of 4-iodo-2-thiomethylpyrimidine and 4-chloro-2-thiomethylpyrimidine) in triethylamine (2 mL) under an argon atmosphere were added tetrakis(triphenylphosphine)palladium(0) (0.044 g. 4%) and copper(I) iodide (0.010 g, 6%), and the mixture was heated at 80-85° C for 0.5 h. Ammonium chloride was added and the mixture was extracted three times with dichloromethane, was dried (magnesium sulfate) and was evaporated. Purification by flash chromatography, eluting with 1:1 ethyl acetate:hexanes, provided *cis*-[4-(3-cyclopentyloxy-4-methoxyphenyl)-4-(2-methylthiopyrimidin-4-ylethynyl)cyclohexan-1-ol] as a yellow oil (0.36 g, 87%). ¹H-NMR (400 MHz, CDCl₃) δ 8.45 (d, J=5.0 Hz, 1 H), 7.15 (d, J=2.3 Hz, 1 H), 7.03 (dd, J=8.5, 2.3 Hz, 1 H), 7.01 (d, J=5.0 Hz, 1 H), 6.84 (d, J=8.5 Hz, 1 H), 4.81 (m, 1 H), 3.84 (s, 3 H), 3.72 (m, 1 H), 2.57 (s, 3 H), 2.14 (br d, J=12 Hz, 2 H), 2.05 (m, 2 H), 1.8-2.0(m, 10 H), 1.6(m, 2 H) ppm.

### 19c) cis-[4-(3-cyclopentyloxy-4-methoxyphenyl)-4-(2-methylsulfonylpyrimidin-4-ylethynyl)cyclohexan-1-ol]

To a solution of *cis*-[4-(3-cyclopentyloxy-4-methoxyphenyl)-4-(2-methylthiopyrimidin-4-ylethynyl)cyclohexan-1-ol] (0.36 g. 0.82 mmol) in chloroform (5mL) at -10° C under an argon atmosphere was dropwise added over 20 min. a solution of 3-chloroperoxybenzoic acid (0.34 g, 1.97 mmol) in chloroform (3 mL). The reaction was stirred 3h at -10° C, then 3 h at room temperature. A second portion of 3-chloroperoxybenzoic acid (0.11 g, 0.62 mmol) in chloroform (1 mL) was added and stirring was continued for 1.5 h. The reaction was quenched with sodium carbonate (5%), was extracted three times with dichloromethane, was dried (potassium carbonate) and was evaporated. Purification by flash chromatography, eluting with 2.5:97.5 methanol:dichloromethane, provided *cis*-[4-(3-cyclopentyloxy-4-methoxyphenyl)-4-(2-methylsulfonylpyrimidin-4-ylethynyl)cyclohexan-1-ol] as a white foam (031 g, 81%), mp 75-77° C. ¹H-NMR (400 MHz, CDCl₃) δ 8.84 (d, J=5.3 Hz, 1 H), 7.54 (d, J=5.3 Hz, 1 H), 7.10 (d, J=2.3 Hz, 1 H), 7.04 (dd, J=8.5, 2.3 Hz, 1 H), 6.85 (d, J=8.5 Hz, 1 H), 4.81 (m, 1 H), 3.85 (s, 3 H). 3.73 (m, 1 H), 3.38 (s, 3 H), 2.20 (br d, J=12 Hz, 2 H), 2.06 (m, 2 H), 1.8 - 2.0 (m, 10 H), 1.6 (m, 2 H) ppm.

### 19d) cis- [4-(2-aminopyrimidin-4-ylethynyl)-4-(3-cyclopentyloxy-4-methoxyphenyl)-cyclohexan-1-ol]

Into a solution of *cis*-[4-(3-cyclopentyloxy-4-methoxyphenyl)-4-(2-methylsulfonylpyrimidin-4-ylethynyl)cyclohexan-1-ol] (0.31 g, 0.66mmol) in methanol (5 mL) at -78° C was condensed liquid ammonia (5 mL). The pressure tube was sealed and the reaction was stirred at room temperature for 2 h. After cooling, the solvents were evaporated. Purification by flash chromatography, cluting with 4:96 methanol:dichloromethane, followed by trituration from dichloromethane-ether-hexanes, provided *cis*-[4-(2-aminopyrimidin-4-ylethynyl)-4-(3-cyclopentyloxy-4-methoxyphenyl)cyclohexan-1-ol] as a white solid (0.19 g, 74%), mp 173-174° C. Anal. (C₂₄H₂₉N₃O₃ · 0.25 H₂O) calcd: C, 69.96; H, 722; N, 10-20; found: C, 69.66; H, 7.10; N, 10.11.

### Reference Example 20

### cis-[4-(2-aminopyrimidin-5-ylethynyl)-4-(3-cyclopentyloxy-4-methoxyphenyl)-cyclohexan-1-ol]

To a solution of *trans*-[4-(3-cyclopentyloxy-4-methoxyphenyl)-4-ethynylcyclohexan-1-ol] (0.20 g. 0.64 mmol) and and 2-amino-5-bromopyrimidine (0.55 g, 3.2 mmol) in piperidine (2 mL) under an argon atmosphere were added tetrakis(triphenyl-phosphine)palladium(0) (0.030 g, 4%), copper(I) iodide (0.006 g, 6%) and a small crystal of triphenylphosphine, and the mixture was heated at 80-85° C for 0.5 h. Water was added and the mixture was extracted three times with dichloromethane, was dried (potassium carbonate) and was evaporated. Purification by two successive flash chromatographies, cluting first with 5:95 methanol:dichloromethane, then with 3:97 methanol:dichloromethane, provided *cis*-[4-(3-cyclopentyloxy-4-methoxyphenyl)-4-(2-aminopyrimidin-5-ylethynyl) cyclohexan-1-ol] as a tan-brown solid (0.076 g, 29%), mp 136-137°C. Anal. (C₂₄H₂₉N₃O₃ · 0.75 H₂O) calcd: C, 68.47; H, 7.30; N, 9.98; found: C, 68.25; H, 7.09; N. 9.78.

### Reference Example 21

### cis-[4-(thiazol-2-ylethynyl)-4-(3-cyclopentyloxy-4-methoxyphenyl)cyclohexan-1-ol]

To a solution of *trans*-[4-(3-cyclopentyloxy-4-methoxyphenyl)-4-ethynylcyclohexan-1-ol] (0.15 g, 0.48 mmol) and and 2-bromothiazole (0.20 mL, 2.4 mmol) in triethylamine (1.5 mL) under an argon atmosphere were added tetrakis(triphenyl phosphine)palladium(0) (0.022 g. 4%), copper(I) iodide (0.006 g, 6%) and a small crystal of triphenylphosphine, and the mixture was heated at 80-85° C for 1 h. Ammonium chloride was added and the mixture was extracted three times with dichloromethane, dried (magnesium sulfate), and evaporated. Purification by two successive flash chromatographies, eluting first with 6:4 ethyl acetate:hexanes, then with 2:98 methanol:dichloromethane, provided *cis*-[4-(3-cyclopentyloxy-4-methoxyphenyl)-4-(2-aminopyrimidin-5-ylethynyl)cyclohexan-1-ol] as an off-white foam (0.13 g. 68%), mp 45-48° C. Anal. (C₂₃H₂₇NO₃S · 0.5 H₂O) calcd: C, 67.95; H. 6.94; N, 3.45; found: C, 67.91; H, 6.76; N, 3.39.

### Reference Example 22

### trans-[4-(3-cyclopentyloxy-4-methoxyphenyl)-4-(pyrid-2-ylethynyl)cyclohexyl-1-amine]

### 22a) cis-[4-(3-cyclopentyloxy-4-methoxyphenyl)-4-ethynylcyclohexyl-1-amine]

To a solution of *trans*-[4-(3-cyclopentyloxy-4-methoxyphenyl)-4-ethynylcyclohexan-1-ol] (1.0 g, 3.18 mmol), phthalimide (0.70 g, 4.77 mmol) and triphenylphosphine (1.25 g, 4.77 mmol) in tetrahydrofuran (32 mL) was dropwise added diethyl azodicarboxylate (0.75 mL, 4.77 mmol), and the solution was stirred under an argon atmosphere at room temperature for 2 h. Evaporation and purification by flash chromatography, eluting with 2:8 ethyl acetate hexanes, provided the intermediate phthalimide (1.43 g) as a waxy white solid, mp 45-52° C. This was dissolved in 2:1 ethanol:tetrahydrofuran (30mL), was treated with hydrazine hydrate (1.55 mL, 32 mmol) and was stirred under an argon atmosphere at room temperature for 4 days. Water was added and the mixture was extracted three times with 10:90 methanol:dichloromethane, was dried (potassium carbonate) and was evaporated. Purification by flash chromatography, eluting with 0.5:5:95 ammonium hydroxide:methanol:dichloromethane, provided *cis*-[4-(3-cyclopentyloxy-4-methoxyphenyl)-4-ethynyl-cyclohexyl-1-amine] as a colorless oil (0.71 g, 72%). ¹H-NMR (400 MHz, CDCl₃) δ 7.17 (d, J=2.2 Hz, 1H), 7.09 (dd, J=8.5, 2.2 Hz, 1H), 6.82 (d, J=8.5 Hz, 1H), 4.81 (m, 1H), 3.82 (s, 3 H), 3.26 (br s, 1H), 2.36 (s, 1H), 2.1-2.2 (m, 4 H), 1.8 - 2.0 (m, 10 H), 1.6, (m, 2H) ppm.

### 22b) cis-[1-tert-butoxycarbonylamino-4-(3-cyclopentyloxy-4-methoxyphenyl)-4-ethynylcyclohexane]

A mixture of *cis*-[4-(3-cyclopentyloxy-4-methoxyphenyl)-4-ethynylcyclohexyl-1-amine] (0.55 g, 1.75 mmol) and di-*tert*-butyldicarbonate (0.42 g, 1.93mmol) in dichloromethane (8mL) was stirred 20 h and was evaporated. Purification by flash chromatography, eluting with 2:8 ethyl acetate:hexanes provided, *cis*-[1-*tert*-butoxycarbonylamino-4-(3-cyclopentyloxy-4-methoxyphenyl)-4-ethynylcyclohexane] as a colorless oil (0.70 g, 97%). ¹H-NMR (400 MHz, CDCl₃) δ 7.13 (d, J=2.2 Hz, 1H), 7.02(dd, J=8.5, 2.2 Hz, 1H), 6.84 (d, J=8.5 Hz, 1H), 4.81 (m, 1H), 4.64 (m, 1 H),3.84 (s, 3 H), 2.36 (s, 1H), 2.05 (m, 2 H), 1.6 - 2.0 (m, 14 H), 1.45, (s, 9 H) ppm.

### 22c) trans-[1-tert-butoxycarbonylamino-4-(3-cyclopentyloxy-4-methoxyphenyl)-4-(pyrid-2-ylethynyl-cyclohexane]

To a solution of *cis* -[1-*tert*-butoxycarbonylamino-4-(3-cyclopentyloxy-4-methoxyphenyl)-4-ethynylcyclohexane] (0.35 g, 0.85 mmol) and 2-bromopyridine (0.80 mL, 8.5 mmol) in piperidine (2.5 mL) under an argon atmosphere were added tetrakis(triphenylphosphine)palladium(0) (0.039 g, 4%), copper(I) iodide (0.010 g, 6%) and a small crystal of triphenylphosphine, and the mixture was heated at 80-85° C for 0.5 h in the dark. Water was added and the mixture was extracted three times with dichloromethane, was dried (magnesium sulfate) and was evaporated. Purification by flash chromatography, eluting with 25:75 ethyl acetate:hexanes provided *trans-*[1-*tert*-butoxycarbonylamino-4-(3-cyclopentyloxy-4-methoxyphenyl)-4-(pyrid-2-ylethynylcyclohexane] as a light yellow solid (0.30 g, 72%), mp 69-70° C. ¹H-NMR (400 MHz, CDCl₃) δ 8.57 (d, J=4 Hz, 1 H), 7.66 (dt, J=8, 4 Hz, 1 H), 7.42 (d, J=8 Hz, 1 H), 7.26 (br, 1 H), 7.17 (d, J=2.2 Hz, 1H), 7.09 (dd, J=8.5, 2.2 Hz, 1H), 6.85 (d. J=8.5 Hz, 1H), 4.82 (m. 1H), 4.64 (br s, 1 H), 3.85 (s, 3 H), 2.2 (m, 2H), 1.8 - 2.1 (m, 12 H), 1.6 (m, 2 H), 1.45, (s, 9 H) ppm.

### 22d) trans-[4-(3-cyclopentyloxy-4-methoxyphenyl)-4-(pyrid-2-ylethynyl)cyclohexyl-1-amine]

To a solution of *trans*-[1-*tert*-butoxycarbonylamino-4-(3-cyclopentyloxy-4-methoxyphenyl)-4-(pyrid-2-ylethynylcyclohexane] (030 g, 0.61mmol) in dichloromethane (5 mL) at 0° C under an argon atmosphere was added trifluoroacetic acid (0.60 mL, 7.89 mmol). The reaction was stirred at room temperature for 6 h, was cooled to 0° C, quenched with sodium bicarbonate, was diluted with water, was extracted with three times dichloromethane, was dried (magnesium sulfate) and was evapotaied. Purification by flash chromatography, cluting with 0.7:7:93 ammonium hydroxide:methanol:dichloromethane provided *trans*-[4-(3-cyclopentyloxy-4-methoxyphenyl)-4-(pyrid-2-ylethynyl)cyclohexyl-1-amine] as a very viscous oil (0.19 g, 82%). ¹H-NMR (400 MHz, CDCl₃) δ 8.59 (d, J=4 Hz, 1 H), 7.63 (dt, J=7.8, 4 Hz, 1 H), 7.41 (d, J=7.8 Hz, 1 H), 7.26 (m, 1 H), 7.22 (m, 2H), 6.81 (d, J=8.5 Hz, 1H), 4.84 (m, 1H), 3.81 (s, 3 H), 3.4 (br s, 1 H), 2.31 (m, 4H), 1.8 - 2.0 (m, 10 H), 1.6 (m, 2 H) ppm. Anal. (C₂₅H₃₀N₂O₂ · 0.5 H₂O) calcd: C, 75.16; H, 7.82; N, 7.01; found: C, 75.42; H, 7.77; N, 6.91.

### Reference Example 23

### trans-[4-(3-cyclopentyloxy-4-methoxyphenyl)-4-(pyrid-2-ylethynyl)cyclohexyl-1-formamide].

To a preparation of acetic formic anhydride (0.035 mL, 0.38 mmol) at 0° C under an argon atmosphere was added a solution of *trans*-[4-(3-cyclopentyloxy-4-methoxyphenyl)-4-(pyrid-2-ylethynyl)cyclohexyl-1-amine] (0.096 g, 0.24 mmol) in tetrahydrofuran (1.5 mL). The mixture was stirred for 3 h at room temperature, was diluted with dichloromethane, was washed with sodium bicarbonate and water, was dried (magnesium sulfate) and was evaporated. Purification by flash chromatograhy, eluting with 5:95 methanol:dichloromethane, provided *trans*-[4-(3-cyclopentyloxy-4-methoxyphenyl)-4-(pyrid-2-ylethynyl)cyclohexyl-1-formamide] (which contains a trace of acetamide) as a white foam (0.08 g, 79%), mp 75-76° C. AnaL (C₂₆H₃₀N₂O₃·0.375 H₂O) calcd: C, 73.43; H, 7.26; N, 6.59; found: C, 73.46; H, 7.29; N, 6.25.

### Reference Example 24

### trans-[4-(3-cyclopentyloxy-4-methoxyphenyl)-4-[5-(5-methyl-[1,2,4]oxadiazol-2-yl)thien-2-ylethynyl]cyclohexyl-1-amine], cyclohexylsulfamate salt

### 24a) 2-bromo-5-(5-methyl-[1,2,4]oxadiazol-2-yl)thiophene

2-Bromo-5-(5-methyl-[1,2,4]oxadiazol-2-yl)thiophene was prepared by standard chemistry well known to those versed in the art and was a white solid, mp 48-49° C.

### 24b) trans-[1-tert-butoxycarbonylamino-4-(3-cyclopentyloxy-4-methoxyphenyl)-4-[5-(5-methyl-[1,2,4]oxadiazol-2-yl)thien-2-ylethynyl]cyclohexane]

To a solution of *cis*-[1-*tert*-butoxycarbonylamino-4-(3-cyclopentyloxy-4-methoxyphenyl)-4-ethynylcyclohexane] (0.30 g, 0.73 mmol) and 2-bromo-5-(5-methyl-[1,2,4]oxadiazol-2-yl)thiophene (0.18 g, 0.73 mmol) in triethylamine (5 mL) under an argon atmosphere were added tetrakis(triphenylphosphine)palladium(0) (0.033 g, 4%) and copper(I) iodide (0.008 g, 6%) and a small crystal of triphenylphosphine, and the mixture was heated at 80-85° C for 1 h. Water was added and the mixture was extracted three times with dichloromethane, was dried (magnesium sulfate) and was evaporated. Purification by flash chromatography, eluting with 2:8 ethyl acetate:hexanes, followed by trituration from dichloromethane-hexanes, provided *trans*-[1-*tert*-butoxycarbonylamino-4-(3-cyclopentyloxy-4-methoxyphenyl)-4-[5-(5-methyl-[1,2,4]oxadiazol-2-yl)thien-2-ylethynyl]cyclohexane] as a yellow foam (0.38 g), containing - 40 % of 1,4-*bis*-{[*t*-4-(3-cyclopentyloxy-4-methoxyphenyl)-*r*-1-cyclohexyl-1-amine]-4-yl}buta-1,3-diyne by ¹H-NMR.

### 24c) trans-[4-(3-cyclopentyloxy-4-methoxyphenyl)-4-[5-(5-methyl-[1,2,4]oxadiazol-2-yl)thien-2-ylethynyl]cyclohexyl-1-amine], cyclohexylsulfamate salt

A solution of *trans*-[1-*tert*-butoxycarbonylamino-4-(3-cyclopentyloxy-4-methoxyphenyl)-4-[5-(5-methyl-[1,2,4]oxadiazol-2-yl)thien-2-ylethynyl]cyclohexane] (0.38 g, containing 40 % of the dimer) in dichloromethane (10 mL) at 0° C under an argon atmosphere was treated with trifluoroacetic acid (0.50 mL, 6,5 mmol) and the mixture was stirred for 24 h at room temperature. The solution was quenched with sodium bicarbonate at 0° C, was diluted with water, was extracted three times with 10:90 methanol:dichloromethane, was dried (potassium carbonate) and was evaporated. Purification by flash chromatogaphy, eluting with 0.5:5:95 ammonium hydroxide:methanol:dichloromethane, provided *trans*-[4-(3-cyclopentyloxy-4-methoxyphenyl)-4-[5-(5-methyl-[1,2,4]oxadiazol-2-yl)thien-2-ylethynyl]cyclohexyl-1-amine] as a glassy solid (0.18 g, 59 %). This was dissolved in acetone (0.5 mL) and added to a solution of cyclohexylsulfamic acid (0.066 g, 0.37 mmol) in acetone (0.5 mL). The salt was isolated and the free amine was recovered. A second chromatography using the same solvent system provided free amine (0.048 g), which was treated with cyclohexylsulfamic acid (0.018 g, 0.10 mmol) in acetone (1 mL). After the addition of ether (20 mL), the precipitate was filtered off to provide *trans*-[4-(3-cyclopentyloxy-4-methoxyphenyl)-4-[5-(5-methyl-[1,2,4]oxadiazol-2-yl)thien-2-ylethynyl]cyclohexyl-1-amine], cyclohexylsulfamate salt (0.043 g, 18%) as a white solid, mp 134-135° C. Anal. (C₃₃H₄₄N₄O₆S₂·0.5 H₂O) calcd: C, 59.52; H, 6.81; N, 8.41; found: C, 59.37; H, 6.71; N, 8.46.

### Reference Example 25

### cis-[4-(3-cyclopentyloxy-4-methoxyphenyl)-4-(pyrid-2-ylethynyl)cyclohexyl-1-amine]

### 25a) cis-[1-tert-butoxycarbonylamino-4-(3-cyclopentyloxy-4-methoxyphenyl)-4-ethynyl-cyclohexane]

A solution of 4-(3-cyclopentyloxy-4-methoxyphenyl)-4-ethynyl-cyclohexan-1-one (0.82g, 2.63 mmol), ammonium acetate (2.03 g, 26 mmol), sodium cyanoborohydride (0.17 g, 2.63 mmol) and several 4Å molecular sieves in methanol (10 mL) was stirred under an argon atmosphere at room temperature for 3 days. Several crystals of methyl orange were added, then hydrogen chloride-saturated methanol to - pH 3. The reaction was made basic with sodium hydroxide (10 %), was extracted with 10:90 methanol:dichloromethane, was dried (potassium carbonate) and was evaporated to provide crude *trans*-[4-(3-cyclopentyloxy-4-methoxyphenyl)-4-ethynylcyclohexyl-1-amine] as a yellow oil (0.88 g. 100 %). A solution of the intermediate in dichloromethane (15 mL) was treated with di-*tert*.-butyldicarbonate (0.63g, 2.89 mmol), was stirred 5 h and was evaporated. Purification by flash chromatography, cluting with 15: 85 ethyl acetate:hexanes, provided *trans*-[1-*tert*-butoxycarbonylamino-4-(3-cyclopentyloxy-4-methoxyphenyl)-4-ethynylcyclohexane] as a white foam (0.57 g, 53%, ¹H-NMR shows ~ 20 % *cis* isomer), mp.39-43° C. ¹H-NMR (400 MHz, CDCl₃) δ 7.11 (d, J=2.2 Hz, 1H), 7.04 (dd, J=8.5, 2.2 Hz, 1H), 6.82 (d, 7=8.5 Hz, 1H), 4.80 (m, 1H), 4.6 (m, 0.2 H), 4.5 (m, 0.8 H), 4.0 (m, 0.2 H), 3.84 (s, 0.6 H), 3.83 (s, 2.4 H), 2.43 (s, 0.8 H), 2.36 (s, 0.2 H), 1.6 - 2.1 (m, 16 H), 1.46, (s, 9 H) ppm.

### 25b) cis-[1-tert-butoxycarbonylamino-4-(3-cyclopentyloxy-4-methoxyphenyl)-4-(pyrid-2-ylethynylcyclohexane

To a solution of *trans*-[1-*tert*-butoxycarbonylamino-4-(3-cyclopentyloxy-4-methoxyphenyl)-4-ethynylcyclohexane] (0.45 g. 1.09 mmol) and 2-bromopyridine (1.0 mL. 11 mmol) in piperidine (3 mL) under an argon atmosphere were added tetrakis(triphenylphosphine)palladium(0) (0.050 g, 4%), copper(I) iodide (0.012 g, 6%) and a small crystal of triphenylphosphine, and the mixture was heated at 80-85° C for 0.5 h in the dark. Water was added and the mixture was extracted three times with dichloromethane, was dried (magnesium sulfate) and was evaporated. Purification by flash chromatography, eluting with 2:8 ethyl acetate:hexanes, provided *cis*-[1-*tert*-butoxycarbonylamino-4-(3-cyclopentyloxy-4-methoxyphenyl)-4-(pyrid-2-ylethynylcyclohexane] as a yellow foam (0.41 g, 78%, contains ~ 35 % trans isomer by ¹H-NMR), mp 40-43° C. ¹H-NMR (400 MHz, CDCl₃) δ 8.4 (m , 1 H), 7.65 (m, 1 H), 7.4 (d, 1 H), 7.1 (m, 3 H), 6.85 (m, 1 H), 4.8 (m, 1H), 4.6 (m, 0.65 H), 3.85 (s, 1 H), 3.84 (s, 2 H), 3.55 (m. 0.65 H), 1.5 - 2.2 (m, 16 H), 1.45, (s, 9 H) ppm.

### 25c) cis-[4-(3-cyclopentyloxy-4-methoxyphenyl)-4-(pyrid-2-ylethynyl)cyclohexyl-1-amine]

To a solution of *cis*-[1-*tert*-butoxycarbonylamino-4-(3-cyclopentyloxy-4-methoxyphenyl)-4-(pyrid-2-ylethynylcyclohexane] (0.41 g, 0.84 mmol) in dichloromethane (5 mL) at 0° C under an argon atmosphere was added trifluoroacetic acid (0.65 mL, 8.4 mmol). The reaction was stirred at room temperature for 20 h, was cooled to 0° C, was quenched with sodium bicarbonate, was diluted with water, was extracted twice with 10:90 methanol:dichloromethane, was dried (potassium carbonate) and was evaporated. Purification by flash chromatography, cluting with 0.5:5:95 ammonium hydroxide:methanol:dichloromethane, followed by trituration from ether, provided *cis*-[4-(3-cyclopentyloxy-4-methoxyphenyl)-4-(pyrid-2-ylethynyl)cyclohexyl-1-amine] as a white solid (0.23 g, 69%, containing ~ 20 % of the *trans* isomer), mp 78-80° C. Anal. (C₂₅H₃₀N₂O₂) calcd: C, 66.06; H, 6.65; N, 6.16; found: C, 65.73; H, 6.96; N, 5.98.

### Reference Example 26

### cis-[4-(3-cyclopentyloxy-4-methoxyphenyl)-4-(pyrid-2-ylethynyl)cyclohexyl-1-formamide]

To a preparation of acetic formic anhydride (0.057 mL, 0.64 mmol) at 0° C under an argon atmosphere was added a solution of *cis*-[4-(3-cyclopentyloxy-4-methoxyphenyl)-4-(pyrid-2-ylethynyl)cyclohexyl-1-amine] (0.16 g, 0.40 mmol) in tetrahydrofuran (1.5 mL). The mixture was stirred for 3 h at room temperature, was diluted with dichloromethane, was washed with sodium bicarbonate and water, was dried (magnesium sulfate) and was evaporated. Purification by flash chromatograhy, cluting with 5:95 methanol:dichloromethane, provided *cis*-[4-(3-cyclopentyloxy-4-methoxyphenyl)-4-(pyrid-2-ylethynyl)cyclohexyl-1-formamide] (which contains a trace of acetamide) as a white foam (0.08 g, 79%), mp 80-81° C. Anal. (C₂₆H₃₀N₂O₃·0.375 H₂O) calcd: C, 73.43; H, 7.26; N, 6.59; found: C, 73.46; H, 7.29; N, 6.25.

### Reference Example 27

### cis-[4-(3-cyclopentyloxy-4-methoxyphenyl)-4-[5-(5-methyl-[1,2,4]oxadiazol-2-yl)thien-2-ylethynyl]cyclohexyl-1-amine], cyclohexylsulfamate salt

### 27a) 2-bromo-5-(5-methyl-[1,2,4]oxadiazol-2-yl)thiophene

2-Bromo-5-(5-methyl-[1,2,4]oxadiazol-2-yl)thiophene was prepared by standard chemistry well known to those versed in the art and is a white solid, mp 48-49° C.

### 27b) cis-[1-tert-butoxycarbonylamino-4-(3-cyclopentyloxy-4-methoxyphenyl)-4-[5-(5-methyl-[1,2,4]oxadiazol-2-yl)thien-2-ylethynyl]cyclohexane]

To a solution of *trans*-[4-(3-cyclopentyloxy-4-methoxyphenyl)-4-ethynylcyclohexyl-1-amine] (0.21 g, 0.52 mmol) and 2-bromo-5-(5-methyl-[1,2,4]oxadiazol-2-yl)thiophene (0.13 g, 0.52 mmol) in triethylamine (5 mL) under an argon atmosphere were added tetrakis(triphenylphosphine)palladium(0) (0.024 g, 4%), copper(I) iodide (0.006 g, 6%) and a small crystal of triphenylphosphine, and the mixture was heated at 80-85° C for 1 h. Ammonium chloride was added and the mixture was extracted three times with dichloromethane, was dried (magnesium sulfate) and was evaporated. Purification by two successive flash chromatographies, during first with 2:8 ethyl acetate:hexanes, then with 2:8 acetone:hexanes, provided *cis*-[1-*tert*-butoxycarbonylamino-4-(3-cyclopentyloxy-4-methoxyphenyl)-4-[5-(5-methyl-[1,2,4]oxadiazol-2-yl)thien-2-ylethynyl]cyclohexane] as a white foam (0.20 g, 69%, contains ∼ 20 % dimer impurity by ¹H-NMR), mp 60-68° C.

### 27c) cis-[4-(3-cyclopentyloxy-4-methoxyphenyl)-4-[5-(5-methyl-[1,2,4]oxadiazol-2-yl)thien-2-ylethynyl]cyclohexyl-1-amine], cyclohexylsulfamate sah

A solution of *cis*-[1-*tert*-butoxycarbonylamino-4-(3-cyclopentyloxy-4-methoxyphenyl)-4-[5-(5-methyl-[1,2,4]oxadiazol-2-yl)thien-2-ylethynyl]cyclohexane] (0.21 g, containing 20 % of the dimer) in dichloromethane (5 mL) at 0° C under an argon atmosphere was treated with trifluoroacetic acid (0.28 mL, 3.6 mmol) and was stirred 24 h at room temperature. The solution was quenched with sodium bicarbonate at 0° C, was diluted with water, was extracted three times with 10:90 methanol:dichloromethane, was dried (potassium carbonate) and was evaporated. Purification by flash chromatogaphy, eluting with 0.3:3:97 ammonium hydroxide:methanol:dichloromethane, provided *cis*-[4-(3-cyclopentyloxy-4-methoxyphenyl)-4-[5-(5-methyl-[1,2,4]oxadiazol-2-yl)thien-2-ylethynyl]cyclohexyl-1-amine] as a colorless glass (0.11 g, 0.24 mmol, 69%). This intermediate was dissolved in acetone (0.5 mL) and was added to a solution of cyclohexylsulfamic acid (0.045 g, 0.24 mmol) in acetone (1 mL). After the addition of ether, the precipitate was filtered off to provide *cis*-[4-(3-cyclopentyloxy-4-methoxyphenyl)-4-[5-(5-methyl-[1,2.4]oxadiazol-2-yl)thien-2-ylethynyl]cyclohexyl-1-amine], cyclohexylsulfamate salt (0.14 g, 58%) as a white solid, mp 152-154° C. Anal. (C₃₃H₄₄N₄O₆S₂) calcd: C, 60.34; H, 6.75; N, 8.53; found: C, 60.01; H, 6.63; N, 8.32.

### Reference Example 28

### cis-[4-(3-cyclopentyloxy-4-methoxyphenyl)-4-(2-[3-(3-methyl[1,2,4]oxadiazol-5-yl)phenyl]ethynyl)cyclohexan-1-ol].

### 28a) 5-(3-iodophenyl)-3-methyl[1,2,4]oxadiazole

5-(3-Iodophenyl)-3-methyl[1,2,4]oxadiazole was prepared by standard chemistry well known to those versed in the art and was a white solid, mp 102-103° C.

### 28b) cis-[4-(3-cyclopentyloxy-4-methoxyphenyl)-4-(2-[3-(3-methyl[1,2,4]oxadiazol-5-yl)phenyl]ethynyl)cyclohexan-1-ol]

To a solution of *trans*-[4-(3-cyclopentyloxy-4-methoxyphenyl)-4-ethynylcyclohexan-1-ol] (0.11 g, 0.35 mmol) and 5-(3-iodophenyl)-3-methyl[1,2,4]oxadiazole (0.15 g, 0.52 mmol) in triethylamine (4 mL) under an argon atmosphere were added tetrakis(triphenylphosphine)palladium (0.017 g, 0.015 mmol), copper(I) iodide (0.004 g, 0.021 mmol) and a small crystal of triphenylphosphine. After heating the mixture at 70° C for 1.5 h., the reaction was quenched by addition of aqueous ammonium chloride solution, and the solvent was concentrated. The mixture was extracted three times with methylene chloride, and the organic phase was washed with water, was dried (sodium sulfate) and was evaporated. Purification by flash chromatography, eluting with 45:55 ethyl acetate:hexanes and crystallizing from ethyl ether, provided *cis*-[4-(3-cyclopentyloxy-4-methoxyphenyl)-4-(2-[3-(3-methyl[1,2,4]oxadiazol-5-yl)phenyl]ethynyl)cyclohexan-1-ol] as a white solid (0.144 g, 87%), mp 71.5-73.5 °C. Anal. (C₂₉H₃₂N₂O₄) calcd: C, 73.71; H, 6.83, N, 5.93 found: C, 73.60, H, 6.91, N, 5.76.

### Reference Example 29

### cis-[4-(3-cyclopentyloxy-4-methoxyphenyl)-4-(2-[3-(5-methyl[1,3,4]oxadiazol-2-yl)phenyl]ethynyl)cyclohexan-1-ol].

### 29a) 2-(3-iodophenyl)-5-methyl[1,3,4]oxadiazole

2-(3-Iodophenyl)-5-methyl[1,3,4]oxadiazole was prepared by standard chemistry well known to those versed in the art and is a white solid, mp 112-113.5 ° C.

### 29b) cis-[4-(3-cyclopentyloxy-4-methoxyphenyl)-4-(2-[3-(5-methyl[1,3,4]oxadiazol-2-yl)phenyl]ethynyl)cyclohexan-1-ol]

To a solution of *trans*-[4-(3-cyclopentyloxy-4-methoxyphenyl)-4-ethynylcyclohexan-1-ol] (0.125 g, 0.40 mmol) and 2-(3-iodophenyl)-5-methyl[1,3,4]oxadiazole (0.171 g, 0.60 mmol) in triethylamine (7 mL) under an argon atmosphere were added tetrakis(triphenylphosphine)palladium (0.020 g, 0.017 mmol), copper(I) iodide (0.0042 g, 0.022 mmol) and a small crystal of triphenylphosphine. After heating the mixture at 75° C for 1.75 h, the reaction was quenched by addition of aqueous ammonium chloride solution, and the solvent was concentrated. The mixture was extracted three times with methylene chloride, and the organic phase was washed with water, was dried (sodium sulfate) and was evaporated. Purification by flash chromatography, eluting with 40 to 50% ethyl acetate in methylene chloride and crystallizing from ethyl ether, provided *cis*-[4-(3-cyclopentyloxy-4-methoxyphenyl)-4-(2-[3-(5-methyl[1,3,4]oxadiazol-2-yl)phenyl]ethynyl)cyclohexan-1-ol] as a white solid (0.119 g, 63%), mp 117.5-119 ° C. Anal. (C₂₉H₃₂N₂O₄·1/8H₂O) calcd: C, 73.36; H, 6.85, N, 5.90 found: C, 73.25; H, 6.94, N, 5.75. ¹H-NMR (400 MHz, CDCl₃) δ 8.12 (s, 1H), 7.98(d-d, J=1.4 Hz; J=7.9 Hz, 1H), 7.60(d-d, J=1.3 Hz, J=7.8 Hz, 1H), 7.46 (t, J=7.8 Hz, 1H), 7.19 (d, J=2.2, 1H), 7.10 (d-d, J=2.1 Hz, J=8.5 Hz, 1H), 6.85 (d, J=8.5 Hz, 1H), 4.81 (p, J=4.3 Hz, 1H), 3.85 (s, 3H), 3.73 (m, 1H), 2.63 (s, 3H), 2.2-1.8 (m, 14H), 1.7-1.5 (m, 7H with H₂O).

### Reference Example 30

### cis-[4-(3-cyclopentyloxy-4-methoxyphenyl)-4-(2-[3-(5-methyl[1,2,4]oxadiazol-3-yl]phenyl]ethynyl)cyclohexan-1-ol],

To a solution of 4-(3-cyclopentyloxy-4-methoxyphenyl)-4-(2-[3-(5-methyl[1,2,4]oxadiazol-3-yl)phenyl]ethynyl)cyclohexan-1-one (0.084 g, 0.18 mmol, prepared as described in co-pending application P50283 filed on even date herewith) in 1,2-dimethoxyethane (3 mL) under an argon atmosphere was added dropwise a solution of sodium borohydride (0.015 g, 0.40 mmol) in 1,2-dimethoxyethane (5 mL). After 2 h stirring at room temperature, the reaction was quenched by addition of aqueous ammonium chloride solution. The solvent was concentrated and the residue was extracted into methylene chloride, was washed with water, was dried (sodium sulfate) and was evaporated. Purification by flash chromatography, cluting with 35% ethyl acetate in hexanes and crystallizing from ethyl ether, provided *cis*-[4-(3-cyclopentyloxy-4-methoxyphenyl)-4-(2-[3-(5-methyl[1,2,4]oxadiazol-3-yl)phenyl]ethynyl)cyclohexan-1-ol] as a white solid (0.050 g, 58%), mp 101-103 °C. Anal. (C₂₉H₃₂N₂O₄.1/5H₂O) calcd: C, 73.15; H, 6.86, N, 5.88 found: C, 73.11; H, 6.85, N, 5.85. ¹H-NMR (400 MHz, CDCl₃) δ 8.18 (s , 1H), 8.00 (d, J=7.9 Hz, 1H), 7.58 (d, J=7.8 Hz, 1H), 7.43 (t, J=7.8 Hz, 1H), 7.19 (d, J=2.2, 1H), 7.10 (d-d, J=2.1 Hz, J=8.5 Hz, 1H), 6.85 (d, J=8.5 Hz, 1H), 4.82 (p, J=43 Hz, 1H), 3.85 (s, 3H), 3.72 (m, 1H), 2.67 (s, 3H), 2.2-1.8 (m, 13H), 1.7-1.5 (m, 6H with H₂O).

### Example 31

### Preparation of cis-4-(3-Cyclopentyloxy-4-methoxyphenyl)-4-[3-(5-trifluoromethyl-[1,2,4]oxadiazol-3-yl)phenylethynyl)cyclohexan-1-ol

### 31a) 3-(3-iodophenyl)-5-trifluoromethyl-[1,2,4]oxadiazole

3-(3-Iodophenyl)-5-trifluoromethyl-[1,2,4]oxadiazole was prepared by standard chemistry well known to those versed in the art and was a white solid, mp 36-37° C.

### 31b) cis-4-(3-Cyclopentyloxy-4-methoxyphenyl)-4-[3-(5-trifluoromethyl-[1,2,4]oxadiazol-3-yl)phenylethynyl)cyclohexan-1-ol

A stirred mixture of *trans*-[4-(3-cyclopentyloxy-4-methoxyphenyl)-4-ethynylcyclohexan-1-ol] (0.22 g, 0.7 mmol) and 3-(3-iodophenyl)-5-trifluoromethyl-[1,2,4]oxadiazole (0.24 g, 0.7 mmol) in dry triethylamine (5 mL) was treated under an argon atmosphere trace tetrakis(triphenylphosphine)palladium and copper(I) iodide. The mixture was refluxed for 0.2 h, was cooled to RT and was evaporated.The residue was adsorbed onto silica gel and was purified by flash chromatography cluting with 1:1 hexanes/ethyl acetate and trituration from hexanes/ ethyl acetate provided the title compound as a white solid (0.26g, 71%), mp 85-86° C

### Example 32

### Preparation of cis-4-(3-cyclopentyloxy-4-methoxyphenyl)-4-[3-(5-methyl-[1,3,4]thiadiazol-2-yl)phenylethynyl]cyclohexan-1-ol

### 32a) 1-iodo-3-(5-methyl-[1,3,4]thiadiazol-2-yl)benzene

1-Iodo-3-(5-methyl-[1,3,4]thiadiazol-2-yl)benzene was prepared by standard chemistry well known to those versed in the art and was white solid, m.p. 86-89° C.

### 32b) 4-(3-cyclopentyloxy-4-methoxyphenyl)-4-[3-(5-methyl-[1,3,4]thiadiazol-2-yl)phenylethynyl]cyclohexan-1-ol

To a solution of *trans*-[4-(3-cyclopemyloxy-4-methoxyphenyl)-4-ethynylcyclohexan-1-ol] (0.15 g, 0.48 mmol) and 1-iodo-3-(5-methyl-[1,3,4]thiadiazol-2-yl)benzene (0.15 g, 0.48 mmol) in triethylamine (5 mL) under an argon atmosphere was added trace tetrakis(triphenylphosphine)palldium(0) and copper(I) iodide. The mixture was refluxed for 0.20 h, was cooled to room temperature and was concentrated *in vacuo*. Purification by flash chromatography, eluting with 1:1 hexanes/ethyl acetate and trituration from hexanes/ethyl acetate provided the title compound as a white solid (0.17g, 74%), m.p. 129-130° C.

### Example 33

### Preparation of trans-[4-(2-acetamidopyrimidin-5-ylethynyl)-4-(3-cyclopentyloy-4-methoxyphenyl)cyclohexan-1-ol]

To a suspension of *cis*-[4-(3-cyclopentyloxy-4-methoxyphenyl)-4-ethynylcyclohexan-1-ol] (0.50 g, 1.59 mmol) and 2-acetamido-5-bromopyrimidine (0.35 g. 1.59 mmol) in triethylamine (7 mL) under an argon atmosphere was added tetrakis(triphenylphosphine)palladium(0) (0.073 g, 4%) and copper(I) iodide (0.019 g, 6%) and the mixture was heated at 80-85° C for 0.75 h. Water was added and the mixture was extracted three times with dichloromethane, was dried (magnesium sulfate) and was evaporated. The residue was pre-adsorbed onto silica and purified by chromatoraphy on silica gel, eluting the nonpolar impurities with 30:70 to 50:50 ethyl acetate:dichloromethane, and eluting the desired product in 60:40:1 ethyl acetate:dichloromethane:methanol. This was crystallized from ethyl acetate:hexanes to provide *trans*-[40(2-acetamidopyrimidin-5-yl-ethynyl)-4-(3-cyclopentyloxy-4-methoxyphenyl)cyclohexan-1-ol] as a white solid (0.47g, 66%), mp 163-164° C. Anal. (C26H31N304 0.25 H20) calcd:VC, 69.47; H, 6.95; n, 9.35; found: C, 69.27; H, 6.98; N, 8.97.

### Example 34

### Preparation of trans-[4-(2-aminopyrimidin-5-yl-ethynyl)-4-(3-cyclopentyloxy-4-methoxyphenyl)-cyclohexan-1-ol

To a solution of sodium methoxide (0.137 g, 2.5 mmol) in dry methanol (3.8 mL) was added *via* cannula a solution of *trans*-[4-(2-acetamidopyrimidin-5-ylethynyl)-4-(3-cyclopentyloxy-4-methoxyphenyl)cyclohexan-1-ol] (0.32 g, 0.71 mmol) in dry methanol (7.6 mL including rinses). After stirring for 3 h at 25°C, the reaction was quenched into cold water, extracted with methylene chloride, filtered, dried (magnesium sulfate) and concentrated *in vacuo*. The resulting solid was recrystallized from ethyl acetate:hexandes and dried *in vacuo* at 80° C to afford the desired product as a white solid (0.154 g, 53%), mp 170.5-172.0°C. Anal. (C24H29N303 0.125 H20) calcd: C, 70.35; H, 7.20; N, 10.25; found: C, 7034; H, 7.18; N, 10.14.

### Example 35

### Preparation of cis-[4-(2-methylaminopyrimidin-5-ylethynyl)-4-(3-cyclopentyloxy-4-methoxyphenyl)cyclohexan-1-ol]

### 35(a) 5-bromo-2-methylacetamidopyrimidine.

A mixture of 2-acetamido-5-bromopyrimidine (0.22g, 1 mmol,) potassium carbonate (0.21g, 1.5 mmol) and iodome thane (0.10mL, 1.6 mmol) in tetrahydrofuran was stirred at room temperature for three days. The mixture was filtered, was evaporated and was purified by flash chromatography, cluted with 2:8 ethyl acetate:hexanes, to provide the desired product as a white solid (0.03g, 13%) ¹H-NMR (CDCl₃, 400 Mhz) δ 8.58 (s, 2H), 3.39 (s, 3H), 2.39 (s, 3H) ppm.

### 37b) cis-[4-(2-methylacetamidopyrimidin-5-yl-ethynyl)-4-(3-cyclopentyloxy-4-methoxyphenyl)-cyclohexan-1-ol].

To a suspension of *trans*-[4-(3-cyclopentyloxy-4-methoxyphenyl)-4-ethynylcyclohexan-1-ol) (0.20 g, 0.64 mmol) and a mixture (-6:4) of 2-acetamido-5-bromopyrimidine and 5-bromo-2-methylacetamidopyrimidine (0.14 g, -0.32 mmol of each component) in triethylamine (5 mL) under an argon atmosphere was added tetrakis(triphenylphosphiine)palladium(0) (0.03 g, 4%) and copper(I) iodide (0.008 g, 6%) and the mixture was heated at 80-85° C for 0.5 h. The reaction was cooled, water was added and the mixture was extracted three times with dichloromethane, was dried (magnesium sulfate) and was evaporated. The residue was purified by chromatography on silica gel with 1:1 and 1:3 ethyl acetate:hexanes, to provide the desired compound as a colorless oil (which contains a trace amount of 2-acetamido-5-bromopyrimidine). ¹H-NMR (CDCl₃, 400 Mhz) δ 8.65 (s, 2H), 7.12 (d. J=2.0 Hz, 1H), 7.07 (dd, J=8.3, 2.0 Hz, 1H), 6.85, d, J=8.3Hz, 1H), 4.80 (m, 1H), 3.85 (s, 3H), 3.83 (m, 1H), 3.49 (s, 3H), 2.48 (s, 3H), 1.8 - 2.2 (m, 14H, 1.65 (m, 2H) ppm.

### 37c) cis-[4-(2-methylaminopyrimidin-5-ylethynyl)-4-(3-cyclopentyloxy-4-methoxyphenyl)-cyclohexan-1-ol]

To a stirred mixture of sodium methoxide (0.025 g, 0.43 mmol) in dry methanol (2 mL) under an argon atmosphere was added a solution of *cis*-4-(2-methylacetamidopyrimidin-5-ylethynyl)-4-(3-cyclopentyyloxy-4-methoxyphenyl)cyclohexan-1-ol (0.07 g, 0.15 mmol). The mixture was refluxed for 0.5 h, was cooled to room temperature, wa diluted with water, was extracted three times with dichloromethane, was dried (magnesium sulfate) and was evaporated. Purification by flash chromatography, eluting with 3:1 ethyl acetate:hexanes, followed by trituration from ethyl acetate/hexanes, provided the title compound as a white solid (0.032 g, 61%), m.p. 118-119°C. Anal. (C25H31N3O3 0.75 H2O) calcd:C, 69.02; H, 7.52; N, 9.66; found: C, 68.84; H, 7.11; N, 9.53.

### Example 36

### Preparation of trans-[4-(2-aminopyrimidin-5-ylethynyl)-4-(3-cyclopentyloxy-4-methoxyphenyl)cyclohexyl-1-amine], cyclohexylsulfamate salt

### 36a) 5-bromo-2-propionamidopyrimidine

5-Bromo-2-propionamidopyrimidine was prepared by standard chemistry well known to those versed in the art and was a white solid, mp 161-164° C.

### 36b) trans-[1-tert-butoxycarbonylamino-4-(3-cyclopentyloxy-4-methoxyphenyl)-4-(2-propionamidopyrimidin-5-ylethynyl-cyclohexane]

To a solution of *cis*-[1-*tert*-butoxycarbonylamino-4-(3-cyclopentyloxy-4-methoxyphenyl)-4-ethynylcyclohexane] (0.29 g, 0.69 mmol) and 5-bromo-2-propionamidopyrimidine (0.16 g, 0.69 mmol) in triethylamine (5 mL) under an argon atmosphere were added tetrakis(triphenylphosphine)palladium(0) (0.032 g. 4%), and copper(I) iodide (0.008 g, 6%) and the mixture was heated at 80-85° C for 0.5 h. Water was added and the mixture was extracted three times with dichloromethane, was dried (magnesium sulfate) and was evaporated. Purification by flash chromatography, cluting with 35:65 ethyl acetate:hexanes, provided *trans*-[1-*tert*-butoxycarbonylamino-4-(3-cyclopentyloxy-4-methoxyphenyl)-4-(2-propionamidopyrimidin-5-ylethynyl)-cyclohexane] as a white solid (0.21 g, 54%), mp 87-89° C. ¹H-NMR (400 MHz, CDCl₃) δ 8.57 (s, 2H), 8.17 (m, 1H), 7.11 (d, J=2.2 Hz, 1H), 7.03 (dd, J=8.5, 2.2 Hz, 1H), 6.86 (d, J=8.5 Hz, 1H), 4.81 (m, 1H), 4.63 (m, 1H), 3.85 (s, 3H), 2.75 (q, J=7.4 Hz, 2H), 1.8 - 2.1 (m, 14H), 1.62 (m, 2H), 1.46, (s, 9H), 1.24 (t, J=7.4 Hz, 3H) ppm.

### 34c) trans-[4-(2-aminopyrimidin-5-ylethynyl)-1-tert-butoxycarbonylamino-4-(3-cyclopentyloxy-4-methoxyphenyl)cyclohexane]

A mixture of *trans*-[1-*tert*-butoxycarbonylamino-4-(3-cyclopentyloxy-4-methoxyphenyl)-4-(2-propionamidopyrimidin-5-ylethynyl)cyclohexane] (0.21 g, 0.37 mmol) and sodium methoxide (0.07g, 1.29 mmol) in methanol (5 mL) was refluxed under an argon atmosphere for 0.5 h, then cooled. Water was added and the reaction was extracted three times with dichloromethane, was dried (magnesium sulfate) and was evaporated to provide pure *trans*-[4-(2-aminopyrimidin-5-ylethynyl)-1-*tert*-butoxycarbonylamino-4-(3-cyclopentyloxy-4-methoxyphenyl)cyclohexane] (0.18 g, 97%) as a white solid. mp 183-185° C. ¹H-NMR (400 MBz, CDCl₃) δ 8.34 (s, 2H), 7.13 (d, J=2.3 Hz, 1H), 7.03 (dd, J=8.5, 2.3 Hz, 1H), 6.86 (d, J=8.5 Hz, 1H), 5.27 (d, J=2.2 Hz, 2H), 4.81 (m, 1H), 4.64 (m, 1H), 3.85 (s, 3H), 1.8 - 2.1 (m, 14H), 1.61 (m, 2H), 1.46 (s, 9H) ppm.

### 34d) trans-[4-(2-aminopyrimidin-5-ylethynyl)-4-(3-cyclopentyloxy-4-methoxyphenyl)cyclohexyl-1-amine], cyclohexylsulfamate salt

To a solution of *trans*-[4-(2-aminopyrimidin-5-ylethynyl)-1-*tert*-butoxycarbonylamino-4-(3-cyclopentyloxy-4-methoxyphenyl)cyclohexane] (0.18 g, 0.35mmol) in dichloromethane (5 mL) at 0° C under an argon atmosphere was added trifluoroacetic acid (0.27 mL, 3.52 mmol). The reaction was stirred at room temperature for 24 h, was cooled to 0° C, was quenched with sodium bicarbonate, was diluted with water, was extracted with three times 10/90 methanol/dichloromethane, was dried (magnesium sulfate) and was evaporated. Purification by flash chromatography, cluting with 0.5:10:90 ammonium hydroxide:methanol:dichloromethane provided *trans*-[4-(2-aminopyrimidin-5-ylethynyl)-4-(3-cyclopentyloxy-4-methoxyphenyl)cyclohexyl-1-amine] as a very viscous, colorless oil (0.13 g, 0.33 mmol, 95%). This intermediate was dissolved in acetone (0.5 mL) and treated with a solution of cyclohexylsulfamic acid (0.059 g, 0.33 mmol) in acetone (1.0 mL). Dilution with ether and filtration, followed by trituration from dichloromethane/hexanes provided the title compound as a white solid, mp >230° C (dec.).

### Example 37

*c**is*-[4-(2-aminopyrimidin-5-ylethynyl)-4-(3-cyclopentyloxy-4-methoxyphenyl)cyclohexyl-1-amine], cyclohexylsulfamate salt *cis*-[4-(2-aminopyrimidin-5-ylethynyl)-4-(3-cyclopentyloxy-4-methoxyphenyl)cyclohexyl-1-amine], cyclohexylsulfamate salt is prepared in the same manner as *trans*-[4-(2-aminopyrimidin-5-ylethynyl)-4-(3-cyclopentyloxy-4-methoxyphenyl)cyclohexyl-1-amine], except starting from *cis* -[1-*tert*-butoxycarbonylamino-4-(3-cyclopentyloxy-4-methoxyphenyl)-4-ethynyl-cyclohexane].

### UTILITY EXAMPLES

### EXAMPLE A

### Inhibitory effect of compounds of Formula (I) on in vitro TNF production by human monocytes

The inhibitory effect of compounds of Formula (I) on *in vitro* TNF production by human monocytes may be determined by the protocol as described in Badger *et al*., EPO published Application 0 411 754 A2, February 6, 1991, and in Hanna, WO 90/15534, December 27, 1990.

### EXAMPLE B

Two models of endotoxic shock have been utilized to determine *in vivo* TNF activity for the compounds of Formula (I). The prototocol used in these models is described in Badger *et al*., EPO published Application 0 411 754 A2, February 6, 1991, and in Hanna, WO 90/15534, December 27, 1990.

The compound of Example 1 herein demonstrated a positive *in vivo* response in reducing serum levels of TNF induced by the injection of endotoxin.

### EXAMPLE C

### Isolation of PDE Isozymes

The phosphodiesterase inhibitory activity and selectivity of the compounds of Formula (I) can be determined using a battery of five distinct PDE isozymes. The tissues used as sources of the different isozymes are as follows: 1) PDE Ib, porcine aorta; 2) PDE Ic, guinea-pig heart; 3) PDE III, guinea-pig heart; 4) PDE IV, human monocyte; and 5) PDE V (also called "Ia"), canine trachealis. PDEs Ia, Ib, Ic and III are partially purified using standard chromatographic techniques [Torphy and Cieslinski, MoL Pharmacol., 37:206-214, 1990]. PDE IV is purified to kinetic homogeneity by the sequential use of anion-exchange followed by heparin-Sepharose chromatography [Torphy *et al*., J. Biol. Chem., 267:1798-1804, 1992].

Phosphodiesterase activity is assayed as described in the protocol of Torphy and Cieslinski, MoL Pharmacol., 37:206-214, 1990. Positive IC₅₀'s in the nanomolar to µM range for compounds of the workings examples described herein for Formula (I) have been demonstrated.

## Claims

1. A compound selected from the list consisting of:
*cis*-[4-(3-cyclopentyloxy-4-methoxyphenyl)-4-(4-pyridylethynyl)-cyclohexan-1-ol];
*cis*-4-(3-cyclopentyloxy-4-methoxyphenyl)-4-[3-(5-trifluoromethyl-[1,2,4]oxadiazol-3-yl)phenylethynyl]cyclohexan-1-ol;
*cis*-4-(3-cyclopentyloxy-4-methoxyphenyl)-4-[3-(5-methyl-[1,3,4]thiadiazol-2-yl)phenylethynyl]cyclohexan-1-ol;
*trans*-[4-(2-acetamidopyrimidin-5-ylethynyl)-4-(3-cyclopentyloxy-4-methoxyphenyl)cyclohexan-1-ol];
*trans*-[4-(2-aminopyrimidin-5-yl-ethynyl)-4-(3-cyclopentyloxy-4-methoxyphenyl)-cyclohexan-1-ol], and;
*cis*-[4-(2-methylaminopyrimidin-5-ylethynyl)-4-(3-cyclopentyloxy-4-methoxyphenyl)cyclohexan-1-ol];
and the pharmaceutically acceptable salts thereof.

2. A compound selected from the list consisting of:
*trans*-[4-(2-aminopyrimidin-5-ylethynyl)-4-(3-cyclopentyloxy-4-methoxyphenyl)cyclohexyl-1-amine];
*trans*-[4-(2-ammopyrimidin-5-ylethynyl)-4-(3-cyclopentyloxy-4-methoxyphenyl)cyclohexyl-1-amine], cyclohexylsulfamate salt, and;
*cis*-[4-(2-aminopyrimidin-5-ylethynyl)-4-(3-cyclopentyloxy-4-methoxyphenyl)cyclohexyl-1-amine], cyclohexylsulfamate salt;
and the pharmaceutically acceptable salts thereof.

3. A compound selected from the list defined in claim 2 which is *trans*-[4-(2-aminopyrimidin-5-ylethynyl)-4-(3-cyclopentyloxy-4-methoxyphenyl)cyclohexyl-1-amine], and the pharmaceutically acceptable salts thereof.

4. A compound selected from the list defined in claim 2 which is *trans*-[4-(2-aminopyrimidin-5-ylethynyl)-4-(3-cyclopentyloxy-4-methoxyphenyl)cyclohexyl-1-amine], cyclohexylsulfamate salt.

5. A process for the preparation of a compound as listed in claim 1, which process comprises the reaction of a compound of formula 1A 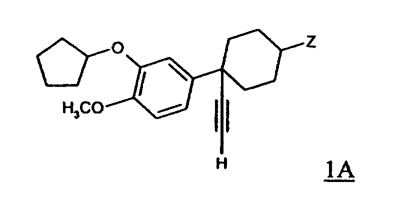 wherein Z is a hydroxyl group or a group convertible thereto, with a compound of formula R₃X, wherein R₃ is selected from 4-pyridyl, 3-(5-trifluoromethyl-[1,2,4]oxadiazol-3-yl)phenyl, 3-(5-methyl-[1,3,4]thiadiazol-2-yl)phenyl, 2-acetamidopyrimidin-5-yl, 2-aminopyrimidin-5-yl, and 2-methylaminopyrimidin-5-yl, and X is a halide.

6. A process for the preparation of a compound as listed in claim 1, which process comprises the reduction of a compound of formula 1B 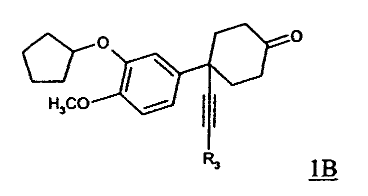 wherein R₃ is selected from 4-pyridyl, 3-(5-trifluoromethyl-[1,2,4]oxadiazol-3-yl)phenyl, 3-(5-methyl-[1,3,4]thiadiazol-2-yl)phenyl, 2-acetamidopyrimidin-5-yl, 2-aminopyrimidin-5-yl, and 2-methylaminopyrimidin-5-yl, to a compound of formula IC 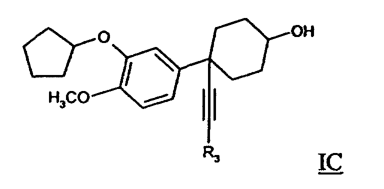 wherein R₃ is selected from 4-pyridyl, 3-(5-trifluoromethyl-[1,2,4]oxadiazol-3-yl)phenyl, 3-(5-methyl-[1,3,4]thiadiazol-2-yl)phenyl, 2-acetamidopyrimidin-5-yl, 2-aminopyrimidin-5-yl, and 2-methylaminopyrimidin-5-yl.

7. A process for the preparation of a compound as listed in claim 2, which process comprises the reaction of a compound of formula 2A 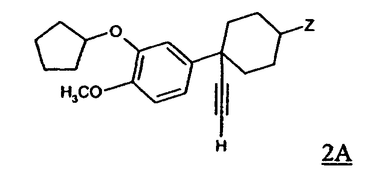 wherein Z is an amino group or a group convertible thereto, with a compound of formula R₃X, wherein R₃ is 2-aminopyrimidin-5-yl and X is a halide, followed by conversion to the cyclohexylsulfamate salt.

8. A pharmaceutical composition comprising a compound as defined in any one of claims 1-4 and a pharmaceutically acceptable carrier or diluent.

9. A compound as defined in any one of claims 1-4 for use in the treatment of allergic and inflammatory disorders.

10. A compound as defined in any one of claims 1-4 for use in the treatment of asthma.

11. A compound as defined in any one of claims 1-4 for use in the inhibition of Tumor Necrosis Factor (TNF).

12. Use of a compound as defined in any one of claims 1-4 for the manufacture of a medicament for the treatment of allergic and inflammatory disorders.

13. Use of a compound as defined in any one of claims 1-4 for the manufacture of a medicament for the treatment of asthma.

## Patentansprüche

1. Verbindung, die aus der Liste ausgewählt ist, die aus:
cis-[4-(3-Cyclopentyloxy-4-methoxyphenyl)-4-(4-pyridylethinyl)-cyclohexan-1-ol],
cis-4-(3-Cyclopentyloxy-4-methoxyphenyl)-4-[3-(5-trifluormethyl-[1,2,4]oxadiazol-3-yl)phenylethinyl]cyclohexan-1-ol,
cis-4-(3-Cyclopentyloxy-4-methoxyphenyl)-4-[3-(5-methyl-[1,3,4]thiadiazol-2-yl)phenylethinyl]cyclohexan-1-ol,
trans-[4-(2-Acetamidopyrimidin-5-ylethinyl)-4-(3-cyclopentyloxy-4-methoxyphenyl)cyclohexan-1-ol],
trans-[4-(2-Aminopyrimidin-5-yl-ethinyl)-4-(3-cyclopentyloxy-4-methoxyphenyl)cyclohexan-1-ol] und
cis-[4-(2-Methylaminopyrimidin-5-ylethinyl)-4-(3-cyclopentyloxy-4-methoxyphenyl)cyclohexan-1-ol] besteht;
und die pharmazeutisch akzeptablen Salze davon.

2. Verbindung, die aus der Liste ausgewählt ist, die aus:
trans-[4-(2-Aminopyrimidin-5-ylethinyl)-4-(3-cyclopentyloxy-4-methoxyphenyl)cyclohexyl-1-amin],
trans-[4-(2-Aminopyrimidin-5-ylethinyl)-4-(3-cyclopentyloxy-4-methoxyphenyl)cyclohexyl-1-amin]-cyclohexylsulfamatsalz und
cis[4-(2-Aminopyrimidin-5-ylethinyl)-4-(3-cyclopentyloxy-4-methoxyphenyl)cyclohexyl-1-amin]-cyclohexylsulfamatsalz besteht;
und die pharmazeutisch akzeptablen Salze davon.

3. Verbindung, die aus der in Anspruch 2 definierten Liste ausgewählt ist, die trans-[4-(2-Aminopyrimidin-5-ylethinyl)-4-(3-cyclopentyloxy-4-methoxyphenyl)cyclohexyl-1-amin] ist, und die pharmazeutisch akzeptablen Salze davon.

4. Verbindung, die aus der in Anspruch 2 definierten Liste ausgewählt ist, die trans-[4-(2-Aminopyrimidin-5-ylethinyl)-4-(3-cyclopentyloxy-4-methoxyphenyl)cyclohexyl-1-amin]-cyclohexylsulfamatsalz ist.

5. Verfahren zur Herstellung einer Verbindung gemäß Anspruch 1, wobei das Verfahren die Reaktion einer Verbindung der Formel (1A) : 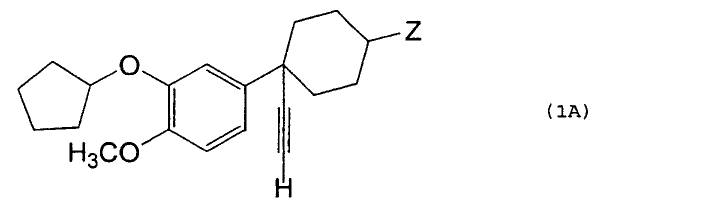 worin Z eine Hydroxylgruppe oder eine dazu umwandelbare Gruppe ist, mit einer Verbindung der Formel R₃X umfaßt, worin R₃ aus 4-Pyridyl, 3-(5-Trifluormethyl-[1,2,4]oxadiazol-3-yl)phenyl, 3-(5-Methyl-[1,3,4]thiadiazol-2-yl)phenyl, 2-Acetamidopyrimidin-5-yl, 2-Aminopyrimidin-5-yl und 2-Methylaminopyrimidin-5-yl ausgewählt ist und X ein Halogenid ist.

6. Verfahren zur Herstellung einer Verbindung gemäß Anspruch 1, wobei das Verfahren die Reduktion einer Verbindung der Formel (1B): 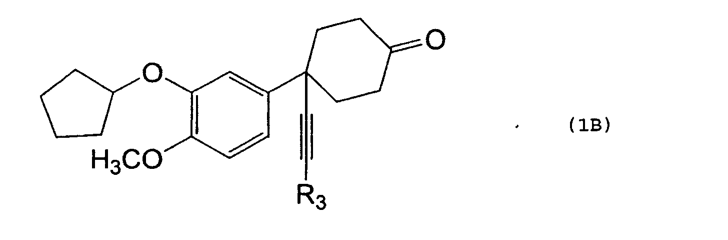 wobei R₃ aus 4-Pyridyl, 3-(5-Trifluormethyl-[1,2,4]oxadiazol-3-yl)phenyl, 3-(5-Methyl-[1,3,4]thiadiazol-2-yl)phenyl, 2-Acetamidopyrimidin-5-yl, 2-Aminopyrimidin-5-yl und 2-Methylaminopyrimidin-5-yl ausgewählt ist, zu einer Verbindung der Formel (1C) umfaßt: 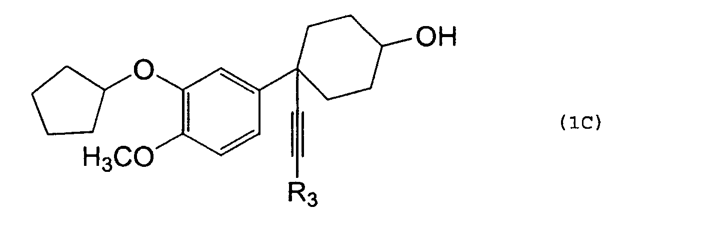 worin R₃ aus 4-Pyridyl, 3-(5-Trifluormethyl-[1,2,4]oxadiazol-3-yl)phenyl, 3-(5-Methyl-[1,3,4]thiadiazol-2-yl)phenyl, 2-Acetamidopyrimidin-5-yl, 2-Aminopyrimidin-5-yl und 2-Methylaminopyrimidin-5-yl ausgewählt ist.

7. Verfahren zur Herstellung einer Verbindung gemäß Anspruch 2, worin das Verfahren die Reaktion einer Verbindung der Formel (2A): 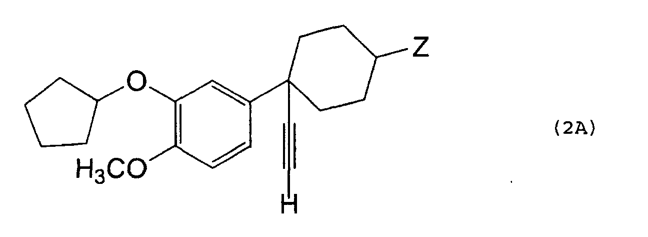 worin Z eine Aminogruppe oder eine dazu umwandelbare Gruppe ist, mit einer Verbindung der Formel R₃X umfaßt, worin R₃ 2-Aminopyrimidin-5-yl ist und X ein Halogenid ist, gefolgt von Umwandlung zum Cyclohexylsulfamatsalz.

8. Pharmazeutische Zusammensetzung, die eine Verbindung gemäß einem der Ansprüche 1 bis 4 und einen pharmazeutisch akzeptablen Träger oder Verdünnungsstoff umfaßt.

9. Verbindung gemäß einem der Ansprüche 1 bis 4 zur Verwendung in der Behandlung von allergischen und inflammatorischen Störungen.

10. Verbindung gemäß einem der Ansprüche 1 bis 4 zur Verwendung in der Behandlung von Asthma.

11. Verbindung gemäß einem der Ansprüche 1 bis 4 zur Verwendung in der Inhibierung von Tumornekrosefaktor (TNF).

12. Verwendung einer Verbindung gemäß einem der Ansprüche 1 bis 4 zur Herstellung eines Medikaments zur Behandlung von allergischen und inflammatorischen Störungen.

13. Verwendung einer Verbindung gemäß einem der Ansprüche 1 bis 4 zur Herstellung eines Medikaments zur Behandlung von Asthma.

## Revendications

1. Composé choisi dans la liste comprenant :
le cis-[4-(3-cyclopentyloxy-4-méthoxyphényl)-4-(4-pyridyléthynyl)-cyclohexan-1-ol] ; le *cis*-4-(3-cyclopentyloxy-4-méthoxyphényl)-4-[3-(5-trifluorométhyl-[1,2,4]oxadiazol-3-yl)phényléthynyl]cyclohexan-1-ol ; le *cis*-4-(3-cyclopentyloxy-4-méthoxyphényl)-4-[3-(5-méthyl-[1,3,4]thiadiazol-2-yl)phényléthynyl]cyclohexan-1-ol ; le *trans*-[4-(2-acétamidopyrimidin-5-yléthynyl)-4-(3-cyclopentyloxy-4-méthoxyphényl)cyclohexan-1-ol] ; le *trans*-[4-(2-aminopyrimidin-5-yl-éthynyl)-4-(3-cyclopentyloxy-4-méthoxyphényl)cyclohexan-1-ol], et ; le *cis*-[4-(2-méthylaminopyrimidin-5-yléthynyl)-4-(3-cyclopentyloxy-4-méthoxyphényl)cyclohexan-1-ol] ; et leurs sels pharmaceutiquement acceptables.

2. Composé choisi dans la liste comprenant :
la trans-[4-(2-aminopyrimidin-5-yléthynyl)-4-(3-cyclopentyloxy-4-méthoxyphenyl)cyclohexyl-1-amine] ; la *trans*-[4-(2-aminopyrimidin-5-yléthynyl)-4-(3-cyclopentyloxy-4-méthoxyphényl)cyclohexyl-1-amine], le sel cyclohexylsulfamate, et ; la *cis*-[4-(2-aminopyrimidin-5-yléthynyl)-4-(3-cyclopentyloxy-4-méthoxyphenyl)cyclohexyl-1-amine], le sel cyclohexylsulfamate; et leurs sels pharmaceutiquement acceptables.

3. Composé choisi dans la liste définie dans la revendication 2 qui est la *trans*-[4-(2-aminopyrimidin-5-yléthynyl)-4-(3-cyclopentyloxy-4-méthoxyphenyl)cyclohexyl-1-amine], et ses sels pharmaceutiquement acceptables.

4. Composé choisi dans la liste définie dans la revendication 2 qui est la *trans*-[4-(2-aminopyrimidin-5-yléthynyl)-4-(3-cyclopentyloxy-4-méthoxyphenyl)cyclohexyl-1-amine], sel cyclohexylsulfamate.

5. Procédé de préparation d'un composé selon la revendication 1, comprenant la mise en réaction d'un composé de formule 1A 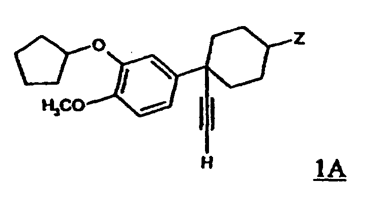 dans laquelle Z est un groupe hydroxyle ou un groupe pouvant être converti en groupe hydroxyle, avec un composé de formule R₃X, dans laquelle R₃ est choisi parmi 4-pyridyle, 3-(5-trifluorométhyl-[1,2,4]oxadiazol-3-yl)phényle, 3-(5-méthyl-[1,3,4,]thiadiazol-2-yl)phényle, 2-acétamidopyrimidin-5-yle, 2-aminopyrimidin-5-yle, et 2-méthylaminopyrimidin-5-yle, et X est un halogénure.

6. Procédé de préparation d'un composé selon la revendication 1, comprenant la réduction d'un composé de formule 1B 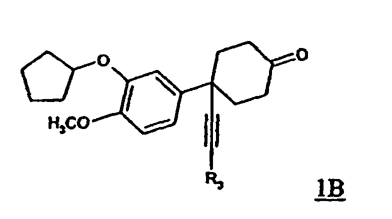 dans laquelle R₃ est choisi parmi 4-pyridyle, 3-(5-trifluorométhyl-[1,2,4]oxadiazol-3-yl)phényle, 3-(5-méthyl-[1,3,4,]thiadiazol-2-yl)phényle, 2-acétamidopyrimidin-5-yle, 2-aminopyrimidin-5-yle, et 2-méthylaminopyrimidin-5-yle, en un composé de formule IC 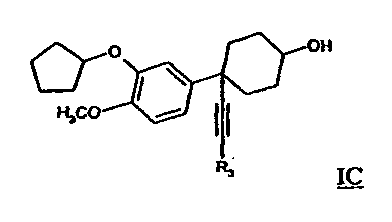 dans laquelle R₃ est choisi parmi 4-pyridyle, 3-(5-trifluorométhyl-[1,2,4]oxadiazol-3-yl)phényle, 3-(5-méthyl-[1,3,4,]thiadiazol-2-yl)phényle, 2-acétamidopyrimidin-5-yle, 2-aminopyrimidin-5-yle, et 2-méthylaminopyrimidin-5-yle.

7. Processus pour la préparation d'un composé comme énuméré dans la revendication 2, lequel processus comprend la mise en réaction d'un composé de formule 2A 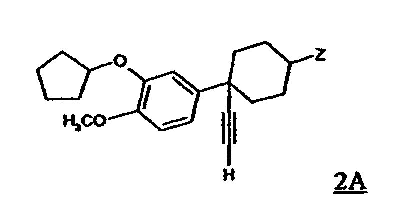 dans laquelle Z est un groupe amino ou un groupe pouvant être converti en groupe amino, avec un composé de formule R₃X, dans laquelle R₃ est 2-aminopyrimidin-5-yle et X est un halogénure, suivie par la conversion en sel cyclohexylsulfamate.

8. Composition pharmaceutique comprenant un composé selon l'une quelconque des revendications 1 à 4 et un porteur ou diluant pharmaceutiquement acceptable.

9. Composé selon l'une quelconque des revendications 1 à 4 destiné à être utilisé dans le traitement de troubles allergiques et inflammatoires.

10. Composé selon l'une quelconque des revendications 1 à 4 destiné à être utilisé pour le traitement de l'asthme.

11. Composé selon l'une quelconque des revendications 1 à 4 destiné à être utilisé pour l'inhibition du facteur de nécrose tumorale (TNF, tumor necrosis factor).

12. Utilisation d'un composé selon l'une quelconque des revendications 1 à 4 pour fabriquer un médicament pour le traitement de troubles allergiques et inflammatoires.

13. Utilisation d'un composé selon l'une quelconque des revendications 1 à 4 pour fabriquer un médicament pour le traitement de l'asthme.
